# EUROPEAN PATENT APPLICATION

(11) **EP 2 275 409 A1**
(43) Date of publication of application: **19.01.2011**
(21) Application number: 10000522.2
(22) Date of filing: 20.01.2010
(51) Int. Cl.: C07D 213/22

(54) **Bipiridine derivative and organic electroluminescence element containing the same**

(30) Priority: 25.06.2009 JP 2009150952; 09.10.2009 JP 2009234702; 14.12.2009 JP 2009282734
(71) Applicant: Yamagata Promotional Organization for Industrial Technology, Yamagata-shi Yamagata 990-2473 (JP)
(72) Inventor: Kimura, Masato, Yamagata-shi Yamagata 990-2473 (JP); Oda, Atsushi, Yamagata-shi Yamagata 990-2473 (JP)
(74) Representative: HOFFMANN EITLE

(57) **Abstract**

The present invention provides a new bipyridine derivative which is suitable for an electron transport material of an organic electronics element and which has bipyridyl central rings as a core, and further provides an organic electroluminescence element containing the derivative.

The bipyridine derivative is expressed by the following general formula. 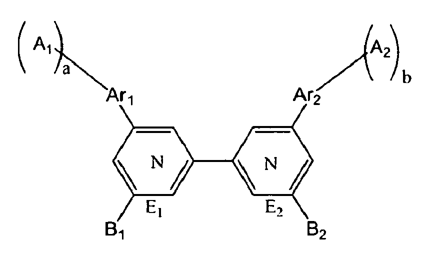 (where A₁, A₂: represent an aromatic heterocyclic group (except for a carbazolyl group) and an aromatic hydrocarbon group and the aromatic heterocyclic group may be combined; a, b = 1 or 2; Ar₁, Ar₂: represent a divalent or trivalent aromatic hydrocarbon group which may be substituted by hydrogen atom, an alkyl group, a cycloalkyl group, or an alkoxy group; B₁, B₂: represent hydrogen atom, an alkyl group, a cycloalkyl group, an alkoxy group, an aromatic hydrocarbon group, or an aromatic heterocyclic group; and ring E₁, ring E₂: represent a pyridine ring, and may have hydrogen atom, an alkyl group, a cycloalkyl group, or an alkoxy group.), which is used as an electron transport material.

## Description

### BACKGROUND OF THE INVENTION

### Field of the Invention

The present invention relates to a material suitable for an organic electronics field, such as an organic solar cell, an organic memory, an organic electroluminescence element (hereinafter abbreviated and referred to as organic EL element), and other devices etc., in particular to a new bipyridine derivative which is useful for an electron transport material and an organic EL element containing the same.

### Description of the Related Art

Since the organic EL element is a self-luminescence type element which contains an organic compound as a light emitting material and allows luminescence at a high speed, it is suitable for displaying a video image, and it has features that allow an element structure to be simple and a display panel to be thin. Having such outstanding features, the organic EL element is spreading in everyday life as a mobile phone display or a vehicle-mounted display.

Further, in recent years, it has attracted attention as nextgeneration lighting, taking advantage of the features of thin plane luminescence as described above.

Such an organic EL element has a basic structure with "anode / light emitting layer/ cathode". In general, a layer is inserted which includes a hole transport material or electron transport material having function to transport holes or electrons in order to attain high efficiency and a long operation life.

Conventionally, Tris(8-quinolinolatoaluminum) (Alq₃) is used as the electron transport material.

However, Alq₃ is a green light-emitting material originally so that green luminescence can be obtained, but it cannot be used in the case of obtaining blue luminescence having a shorter wavelength than that of the green luminescence. Further, Alq₃ has a low ionization potential, and is concerned about decrease in efficiency due to hole penetration.

For this reason, as the other materials having a wide band gap and hole inhibition performance, Japanese Patent Application Publication No. 2008-120696 discloses a tripyridylphenyl derivative as shown in the following (Chemical Formula 1), Japanese Patent Application Publication No. 2007-15993 discloses a triphenylbenzene derivative as shown in the following (Chemical Formula 2), for example. Further, Japanese Patent Application Publication No. 2008-63232 discloses a heteroaryl compound having biphenyl central rings as shown in the following (Chemical Formula 3), and International publication No. WO 2007/026847 discloses a triazole derivative as shown in the following (Chemical Formula 4) .

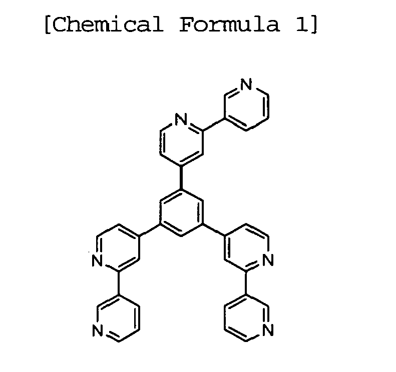

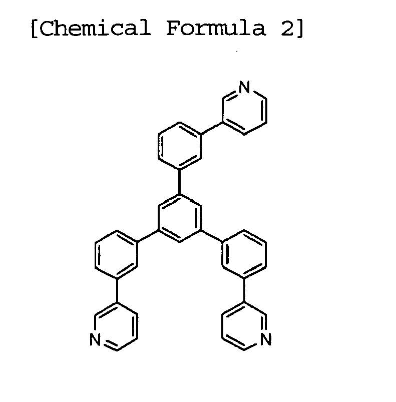

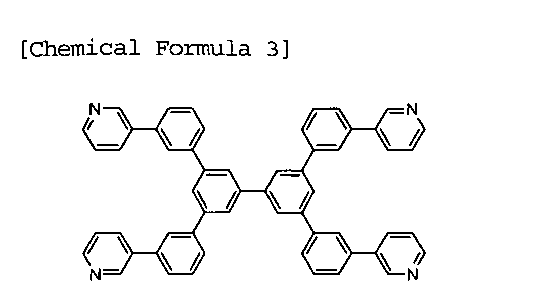

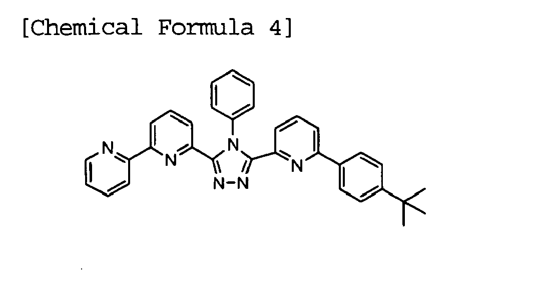

### SUMMARY OF THE INVENTION

The present invention aims to providing a new bipyridine derivative, as an organic electronics element material particularly a material suitable for an electron transport material, which is not disclosed in Japanese Patent Application Publication No. 2008-120696, Japanese Patent Application Publication No. 2007-15993, Japanese Patent Application Publication No. 2008-63232, or International publication No. WO 2007/026847, and which has bipyridyl central rings as a core, and further aims at providing an organic EL element containing the derivative.

According to the present invention, what is expressed by the following general formula (1) is provided as a first bipyridine derivative.

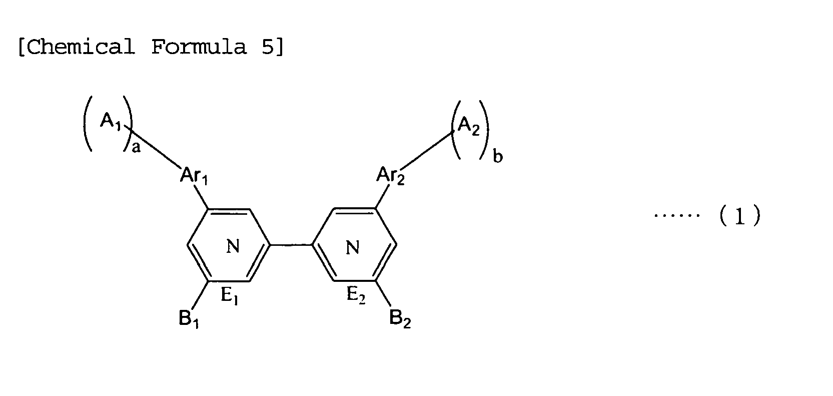

In the above-mentioned general formula (1), A₁ and A₂ each independently represent a substituted or unsubstituted aromatic heterocyclic group (except for a carbazolyl group) and may be a substituent in which an aromatic hydrocarbon group and the aromatic heterocyclic group are combined. a and b are each independently 1 or 2. Ar₁ and Ar₂ each independently represent a divalent or trivalent aromatic hydrocarbon group, and the above-mentioned hydrocarbon group may each be independently substituted by hydrogen atom, an alkyl group, a cycloalkyl group, or an alkoxy group. B₁ and B₂ each independently represent hydrogen atom, an alkyl group, a cycloalkyl group, an alkoxy group, a substituted or unsubstituted aromatic hydrocarbon group, or a substituted or unsubstituted aromatic heterocyclic group. A ring E₁ and a ring E₂ represent a pyridine ring, and may each independently have a substituent selected from hydrogen atom, an alkyl group, a cycloalkyl group, and an alkoxy group.

Of the above-mentioned first bipyridine derivatives, a preferred example may be one that is expressed by the following general formula (2).

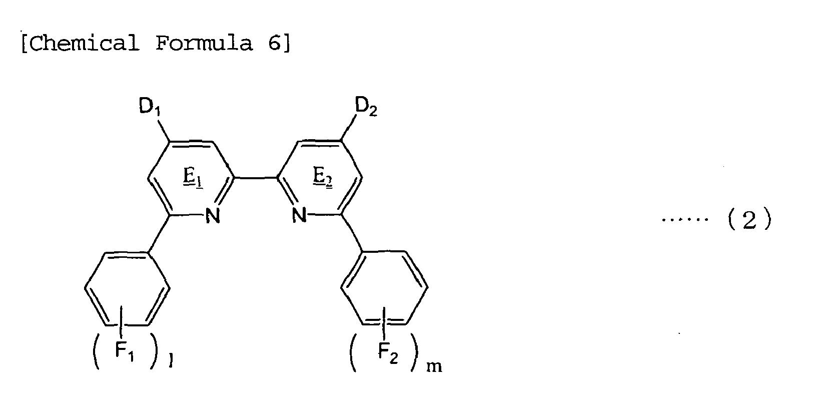

In the above-mentioned general formula (2), F₁ and F₂ each independently represent a substituted or unsubstituted aromatic heterocyclic group (except for a carbazolyl group) and may be a substituent in which an aromatic hydrocarbon group and an aromatic heterocyclic group are combined. Any hydrogen atom on benzene ring may be substituted by an alkyl group, a cycloalkyl group, or an alkoxy group. 1 and m are each independently 1 or 2. D₁ and D₂ each independently represent hydrogen atom, an alkyl group, a cycloalkyl group, an alkoxy group, a substituted or unsubstituted aromatic hydrocarbon group, or a substituted or unsubstituted aromatic heterocyclic group. A ring E₁ and a ring E₂ represent a pyridine ring and may each independently have a substituent selected from hydrogen atom, an alkyl group, a cycloalkyl group, and an alkoxy group.

Further. there may be mentioned one that is expressed by the following general formula (3).

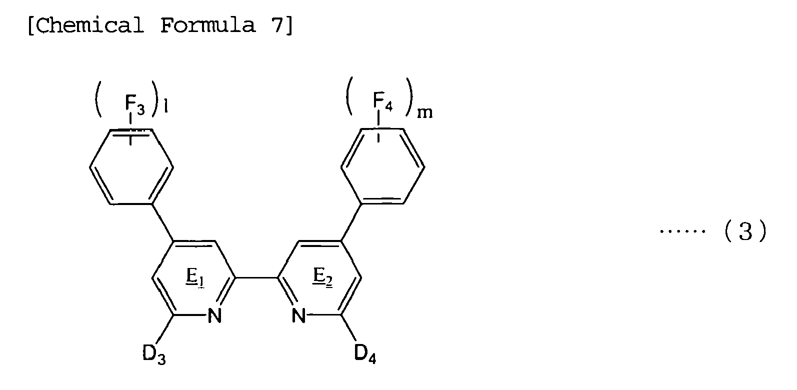

In the above-mentioned general formula (3), F₃ and F₄ each independently represent a substituted or unsubstituted aromatic heterocyclic group (except for a carbazolyl group) and may be a substituent in which an aromatic hydrocarbon group and an aromatic heterocyclic group are combined. Any hydrogen atom on benzene ring may be substituted by an alkyl group, a cycloalkyl group, or an alkoxy group. 1 and m are each independently 1 or 2. D₃ and D₄ each independently represent hydrogen atom, an alkyl group, a cycloalkyl group, an alkoxy group, a substituted or unsubstituted aromatic hydrocarbon group, or a substituted or unsubstituted aromatic heterocyclic group. A ring E₁ and a ring E₂ represent a pyridine ring and may each independently have a substituent selected from hydrogen atom, an alkyl group, a cycloalkyl group, and an alkoxy group.

Further, according to the present invention, what is expressed by the following general formula (4) is provided as a second bipyridine derivative.

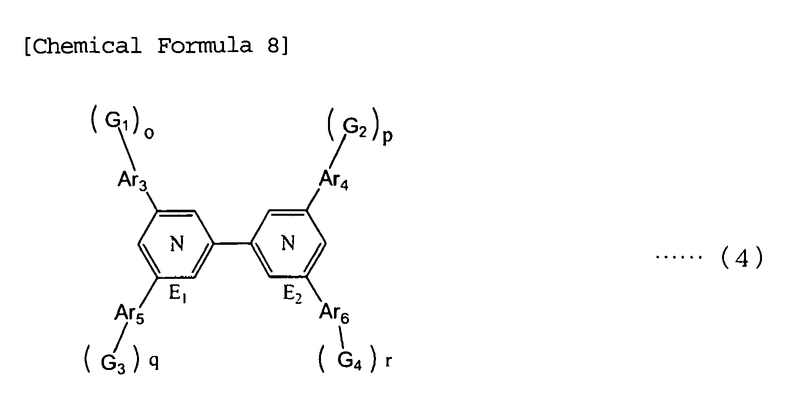

In the above-mentioned general formula (4), G₁-G₄ each independently represent a substituted or unsubstituted aromatic heterocyclic group (except for a carbazolyl group, a phenoxazyl group, and a phenothiazyl group) and may be a substituent in which an aromatic hydrocarbon group and an aromatic heterocyclic group are combined. o, p, q, and r are each independently 1 or 2. Ar₃-Ar₆ each independently represent a divalent or trivalent aromatic hydrocarbon group, and the above-mentioned hydrocarbon group may each be independently substituted by hydrogen atom, an alkyl group, a cycloalkyl group, or an alkoxy group. A ring E₁ and a ring E₂ represent a pyridine ring and may each independently have a substituent selected from hydrogen atom, an alkyl group, a cycloalkyl group, and an alkoxy group.

Of the above-mentioned second bipyridine derivatives, a preferred example may be one that is expressed by the following general formula (5).

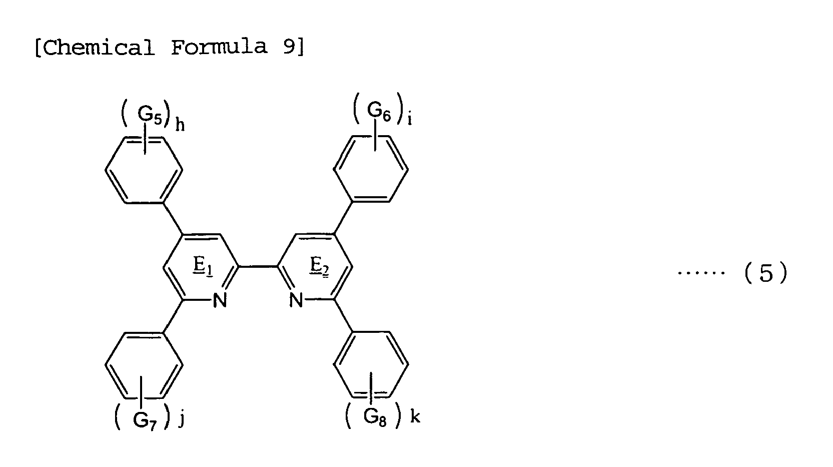

In the above-mentioned general formula (5), G₅-G₈ each independently represent a substituted or unsubstituted aromatic heterocyclic group (except for a carbazolyl group) and may be a substituent in which an aromatic hydrocarbon group and an aromatic heterocyclic group are combined. Any hydrogen atom on benzene ring may be substituted by an alkyl group, a cycloalkyl group, or an alkoxy group. h, i, j, and k are each independently 1 or 2. A ring E₁ and a ring E₂ represent a pyridine ring and may each have independently a substituent selected from hydrogen atom, an alkyl group, a cycloalkyl group, and the alkoxy group.

Further, according to the present invention, what is expressed by the following general formula (6) is provided as a third bipyridine derivative.

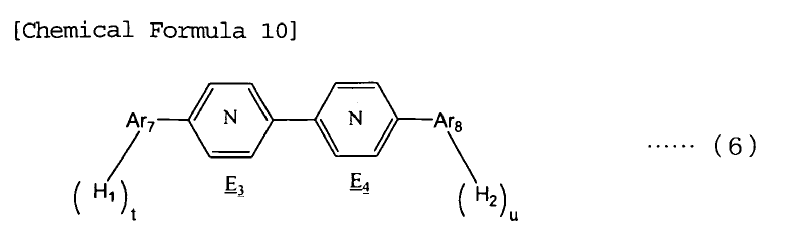

In the above-mentioned general formula (6), H₁ and H₂ are each independently represent a substituted or unsubstituted aromatic heterocyclic group (except for a carbazolyl group) and may be a substituent in which an aromatic hydrocarbon group and an aromatic heterocyclic group are combined. Ar₇ and Ar₈ each independently represent a divalent or trivalent aromatic hydrocarbon group, and the above-mentioned hydrocarbon group may each be independently substituted by hydrogen atom, an alkyl group, a cycloalkyl group, or an alkoxy group. t and u are each independently 1 or 2. A ring E₃ and a ring E₄ represent a pyridine ring, and may each independently have a substituent selected from hydrogen atom, an alkyl group, a cycloalkyl group, and an alkoxy group.

Of the above-mentioned third bipyridine derivatives, a preferred example may be one that is expressed by the following general formula (7).

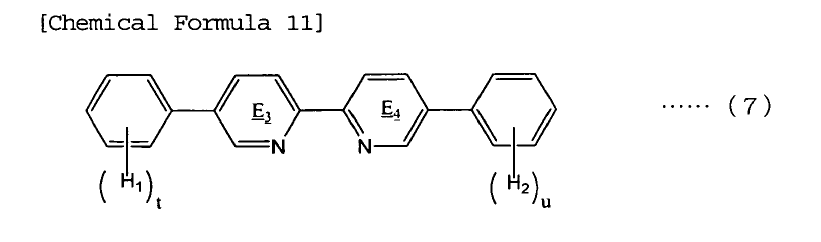

In the above-mentioned general formula (7), H₁ and H₂ each independently represent a substituted or unsubstituted aromatic heterocyclic group (except for a carbazolyl group) and may be a substituent in which an aromatic hydrocarbon group and an aromatic heterocyclic group are combined. Any hydrogen atom on benzene ring may be substituted by an alkyl group, a cycloalkyl group, or an alkoxy group. t and u are each independently 1 or 2. A ring E₃ and a ring E₄ represent a pyridine ring, and may each independently have a substituent selected from hydrogen atom, an alkyl group, a cycloalkyl group, and an alkoxy group.

Further, according to the present invention, what is expressed by the following general formula (8) is provided as a fourth bipyridine derivative.

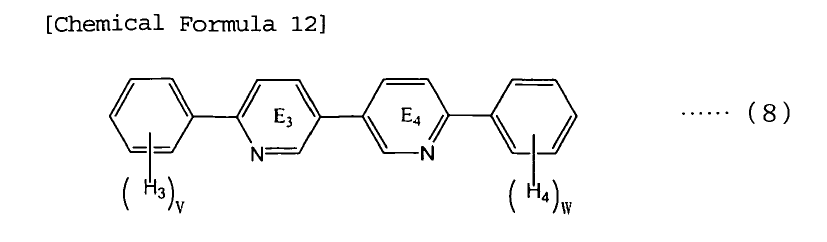

In the above-mentioned general formula (8), H₃ and H₄ each independently represent a substituted or unsubstituted aromatic hydrocarbon group or a substituted or unsubstituted the aromatic heterocyclic group and may be a substituent in which an aromatic hydrocarbon group and an aromatic heterocyclic group are combined. Any hydrogen atom on benzene ring may be substituted by an alkyl group, a cycloalkyl group, or an alkoxy group. v and w are each independently 1 or 2. A ring E₃ and a ring E₄ may each independently have a substituent selected from hydrogen atom, an alkyl group, a cycloalkyl group, and an alkoxy group.

The above-mentioned bipyridine derivative in accordance with the present invention can be used suitably for an organic electronics element material.

As examples of the above-mentioned organic electronics element, there may be mentioned an organic EL element, an organic semiconductor, an organic solar cell, other devices, etc.

Since the above-mentioned bipyridine derivatives are compounds excellent in electron transport performance, they can particularly be used suitably as an electron transport material.

Further, the organic EL element in accordance with the present invention is an organic EL element provided with at least one organic layer between a pair of electrodes, and is **characterized in that** at least one of the above-mentioned organic layers contains any one of the above-mentioned first - fourth bipyridine derivatives.

The above-mentioned bipyridine derivative can demonstrate the above-mentioned electron transport performances in an organic EL element.

Alternatively, the organic EL element in accordance with the present invention is **characterized in that** at least one of an electron transport layer, an electron injection layer, a hole prevention layer, and a light emitting layer contains the above-mentioned first - fourth bipyridine derivatives.

According to the present invention, the new bipyridine derivative having bipyridyl central rings as a core is provided.

The bipyridine derivative in accordance with the present invention is suitable as the organic electronics element material, particularly as the electron transport material, and can suitably be applied to the electron transport layer, the electron injection layer, etc. in the organic EL element.

### BRIEF DESCRIPTION OF THE DRAWINGS

FIG. 1 is a graph showing an emission spectrum of a white phosphorescent element using BP-1p.
FIG. 2 is a graph showing an emission spectrum of the white phosphorescent element using BP-1m.
FIG. 3 is a graph showing an emission spectrum of the white phosphorescent element using BP-2p.
FIG. 4 is a graph showing an emission spectrum of the white phosphorescent element using BP-2m.
FIG. 5 is a graph showing an emission spectrum of the white phosphorescent element using BP-4p.
FIG. 6 is a graph showing an emission spectrum of the white phosphorescent element using BP-4m.
FIG. 7 is a graph showing an emission spectrum of the white phosphorescence element using BP-5p.
FIG. 8 is a graph showing an emission spectrum of the white phosphorescence element using BP-5m.
FIG. 9 is a graph showing an emission spectrum of the white phosphorescence element using BP-6p.
FIG. 10 is a graph showing an emission spectrum of the white phosphorescence element using BP-6m.
FIG. 11 is a graph showing an emission spectrum of a blue phosphorescence element using BP-5p.
FIG. 12 is a graph showing an emission spectrum of the blue phosphorescence element using BP-5m.
FIG. 13 is a graph showing an emission spectrum of the blue phosphorescence element using BP-6p.
FIG. 14 is a graph showing an emission spectrum of the blue phosphorescence element using BP-6m.
FIG. 15 is a graph showing an emission spectrum of the blue phosphorescence element using BP-7p.
FIG. 16 is a graph showing an emission spectrum of the blue phosphorescence element using BP-7m.
FIG. 17 is a graph showing an emission spectrum of the blue phosphorescence element using BMPyPB.
FIG. 18 is a graph showing a current density / voltage characteristic of an electronic element using BP-1 to 4 or BMPyPB.
FIG. 19 is a graph showing a current density / voltage characteristic of the electronics element using BP-5 to 7 or BMPyPB.

### DESCRIPTION OF THE PREFERRED EMBODIMENTS

Hereafter, the present invention will be described in detail.

Bipyridine derivatives in accordance with the present invention are new compounds, in particular four types of bipyridine derivatives expressed by the above-mentioned general formula (1), (4), (6), or (8).

These bipyridine derivatives are compounds which have high electron transport performance.

In the above-mentioned general formulae (1) and (6), A₁, A₂, H₁, and H₂ each independently present a substituted or unsubstituted aromatic heterocyclic group (except for a carbazolyl group). Further, in the general formula (8), H₃ and H₄ each independently represent a substituted or unsubstituted aromatic heterocyclic group or a substituted or unsubstituted the aromatic hydrocarbon group. A₁, A₂, H₁-H₄ may be a monovalent group, having 8-30 carbon atoms, in which an aromatic hydrocarbon group and an aromatic heterocyclic group are combined.

Further, in the above-mentioned general formula (4), G₁-G₄ each independently represent a substituted or unsubstituted aromatic heterocyclic group (except for a carbazolyl group, a phenoxazyl group, and a phenothiazyl group) and may be a monovalent group, having 8-30 carbon atoms, in which an aromatic hydrocarbon group and an aromatic heterocyclic group are combined.

The above-mentioned aromatic heterocyclic group indicates a group which contains either nitrogen, oxygen, or sulfur as a ring member element in addition to carbon (however, such aromatic heterocyclic groups excepted in the general formulae (1), (4), and (6) are not included, respectively). As examples, there may be mentioned 5 or 6 member single rings or fused rings, such as an oxazole ring, an oxadiazole ring, a thiazole ring, a thiadiazole ring, a triazole ring, a pyrazole ring, an imidazole ring, a pyrazine ring, a pyridine ring, a pyridazine ring, a pyrimidine ring, a triazine ring, a quinoline ring, an acridine ring, etc., or monovalent groups which have 2-20 carbon atoms derived from a multi-ring, such as bipyridine in which a plurality of rings are combined.

Further, the above-mentioned aromatic hydrocarbon group indicates a 6 member single ring or fused ring, such as a phenyl group, a naphthyl group, a tetrahydronaphthyl group, an anthranyl group, a fluorenyl group, a biphenyl group, etc., or a monovalent aromatic hydrocarbon group having 6-16 carbon atoms derived from a multi-ring.

The above-mentioned aromatic heterocyclic group and the aromatic hydrocarbon group may each be independently substituted by hydrogen atom, an alkyl group, a cycloalkyl group, or an alkoxy group.

Of these substituents, the alkyl group indicates a saturated hydrocarbon group having 1-8 carbon atoms, such as a methyl group, an ethyl group, a propyl group, a butyl group, etc., which may be of either a straight chain or a branch chain.

The cycloalkyl group indicates a saturated alicyclic hydrocarbon group having 5-12 carbon atoms, such as for example, a cyclohexyl group, a norbornyl group, and an adamantyl group, and they may be either unsubstituted or substituted.

The alkoxy group indicates a saturated aliphatic hydrocarbon group, through ether bonds, such as a methoxy group, a cyclohexyloxy group, etc., which is of a straight chain or of a branch chain and has 1-10 carbon atoms, and a saturated alicyclic hydrocarbon group which has 5-12 carbon atoms and is substituted or unsubstituted.

Hereafter, unless stated otherwise, particular structures of other substituents will not be described further, since they are respectively similar to the above.

B₁ and B₂ each independently represent hydrogen atom, an alkyl group, a cycloalkyl group, an alkoxy group, a substituted or unsubstituted aromatic hydrocarbon group, or a substituted or unsubstituted aromatic heterocyclic group.

The aromatic hydrocarbon group as mentioned herein indicates a 6 member single ring or fused ring, such as a phenyl group, a naphthyl group, a tetrahydronaphthyl group, an anthranyl group, a fluorenyl group, a biphenyl group, etc., or a monovalent aromatic hydrocarbon group or a styryl group having 6-16 carbon atoms derived from a multi-ring.

The above-mentioned aromatic heterocyclic group and the aromatic hydrocarbon group may each be independently substituted by hydrogen atom, an alkyl group, a cycloalkyl group, or an alkoxy group.

a, b, o, p, q, r, t, u, v, and w are each independently 1 or 2.

Ar₁ - Ar₈ each independently represent a divalent or trivalent aromatic hydrocarbon group, and the above-mentioned hydrocarbon group may each be independently substituted by hydrogen atom, an alkyl group, a cycloalkyl group, or an alkoxy group.

The aromatic hydrocarbon group as mentioned herein indicates a 6 member single ring, such as a benzene ring, a naphthalene ring, a tetrahydronaphthalene ring, and an anthracene ring, or a divalent or the trivalent aromatic hydrocarbon group derived from a fused ring having 6-16 carbon atoms.

The rings E₁- E₄ represent a pyridine ring, and may each have independently a substituent selected from hydrogen atom, an alkyl group, a cycloalkyl group, and an alkoxy group.

Hereafter, the same reference signs in the chemical formulae will not be described further, since they are similar to the above.

Of the first bipyridine derivatives expressed by the above-mentioned general formula (1), the compound expressed by the general formula (2) or (3) is preferred more particularly.

In the above-mentioned general formula (2) or (3), F₁-F₄ each independently represent a substituted or unsubstituted aromatic heterocyclic group (except for a carbazolyl group) and may be a substituent in which an aromatic hydrocarbon group and an aromatic heterocyclic group are combined. Any hydrogen atom on benzene ring may be substituted by an alkyl group, a cycloalkyl group, or an alkoxy group. 1 and m are each independently 1 or 2.

D₁-D₄ each independently represent hydrogen atom, an alkyl group, a cycloalkyl group, an alkoxy group, a substituted or unsubstituted aromatic hydrocarbon group, or a substituted or unsubstituted aromatic heterocyclic group.

The aromatic hydrocarbon group as mentioned herein indicates a 6 member single ring or fused ring, such as a phenyl group, a naphthyl group, a tetrahydronaphthyl group, an anthranyl group, a fluorenyl group, a biphenyl group, etc., or a monovalent aromatic hydrocarbon group or a styryl group having 6-16 carbon atoms derived from a multi-ring.

Of the second bipyridine derivatives expressed by the above-mentioned general formula (4), the compound expressed by the above-mentioned general formula (5) is preferred more particularly.

In the above-mentioned general formula (5), G₅-G₈ each independently represent a substituted or unsubstituted aromatic heterocyclic group (except for a carbazolyl group) and may be a substituent in which an aromatic hydrocarbon group and an aromatic heterocyclic group are combined. h, i, j, and k are each independently 1 or 2.

Of the third bipyridine derivatives expressed by the above-mentioned general formula (6), the compound expressed by the above-mentioned general formula (7) is preferred more particularly.

In the above-mentioned general formula (7), any hydrogen atom on benzene ring may be substituted by an alkyl group, a cycloalkyl group, or an alkoxy group.

As for the bipyridine derivative in accordance with the present invention expressed by the above-mentioned general formulae (1) - (8), a structure of a particular compound is shown below.

First, a structure of a general formula (1) is expressed by way of unit notation of Xa and Xb, as shown in the following (Chemical Formula 13). Based on this notation, a particular example of a structure of bipyridine rings is shown in (Chemical Formula 14).

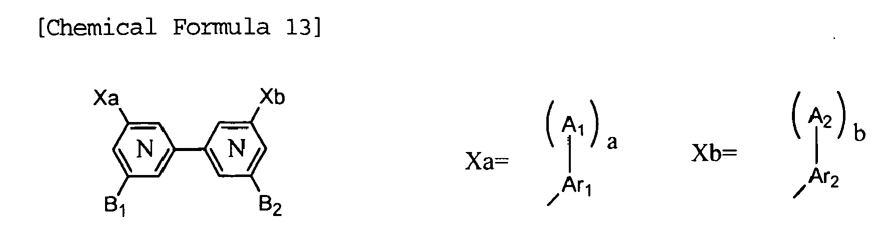

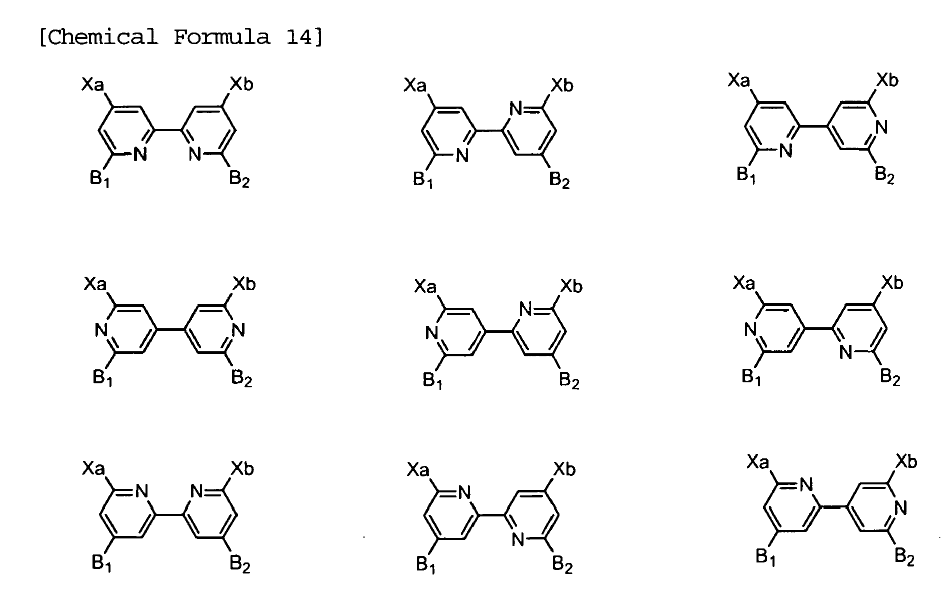

Further, a particular example of a substituent bonded with Xa or Xb and B₁ or B₂ in the above-mentioned Chemical Formula 13 is given below (Chemical Formula 15). In addition, X in (Chemical Formula 15) indicates a position bonded with a pyridine ring of (Chemical Formula 13).

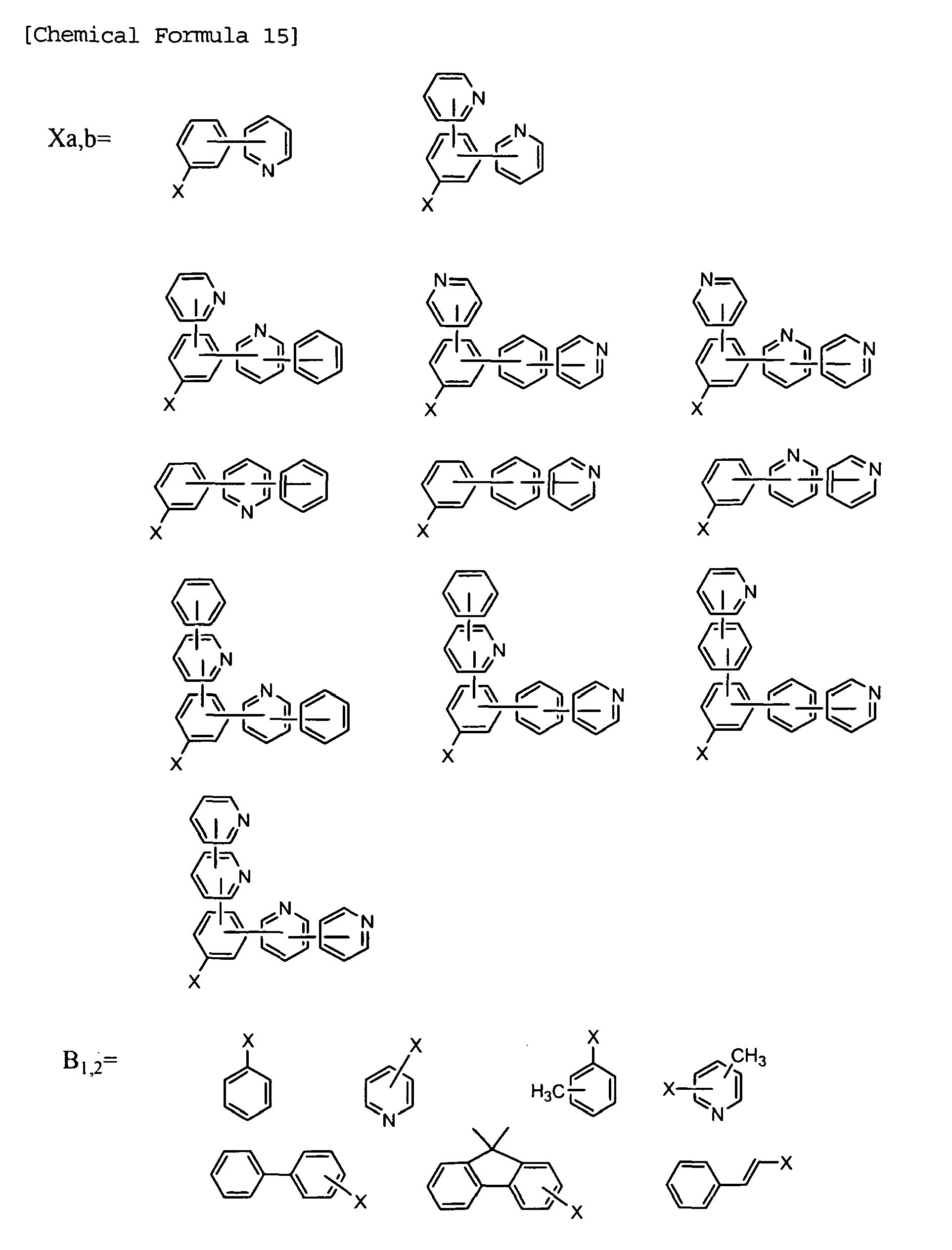

Of the bipyridine derivatives expressed by the above-mentioned general formulae (1) - (3), a structure of a particular compound is illustrated below.

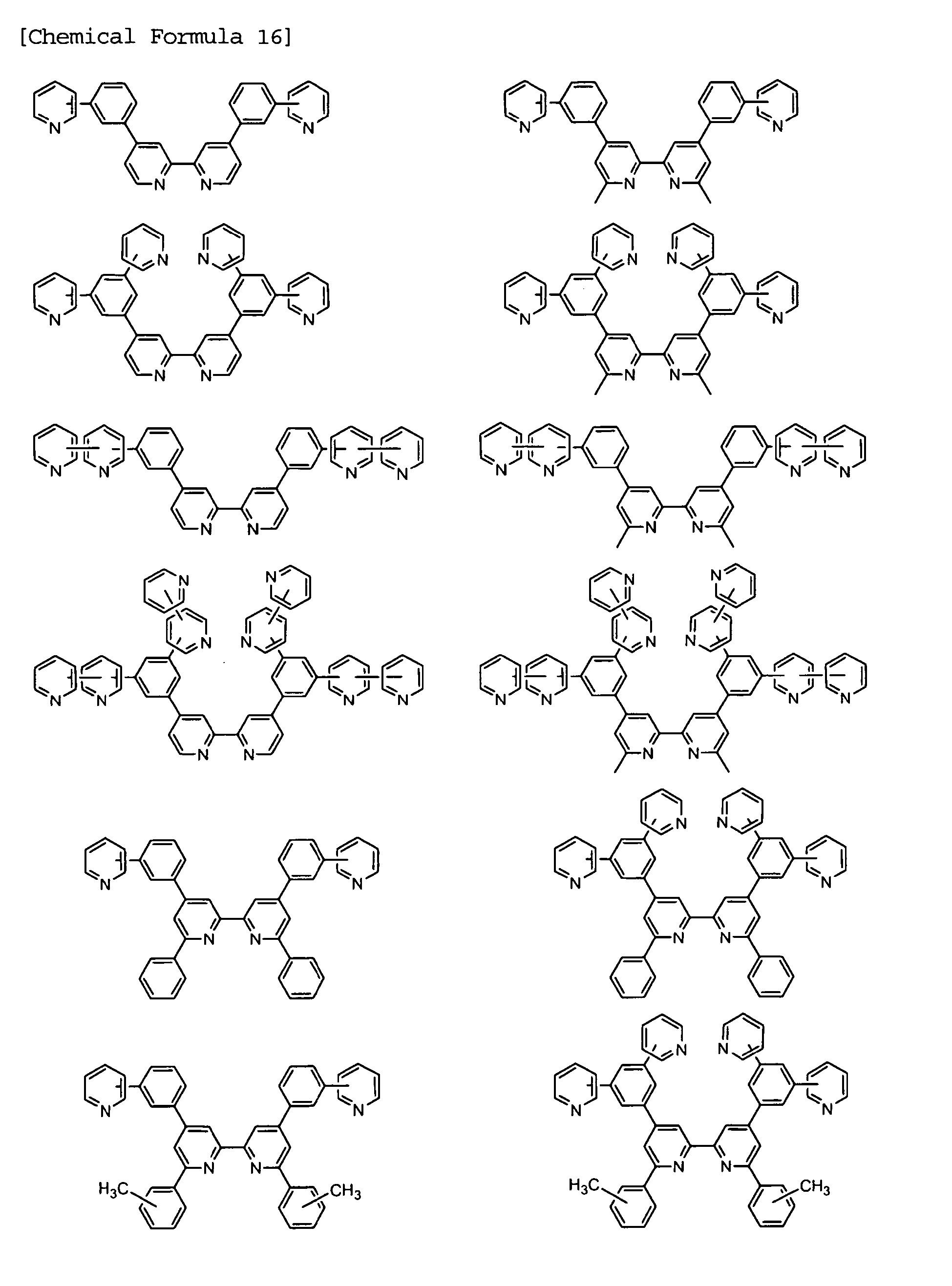

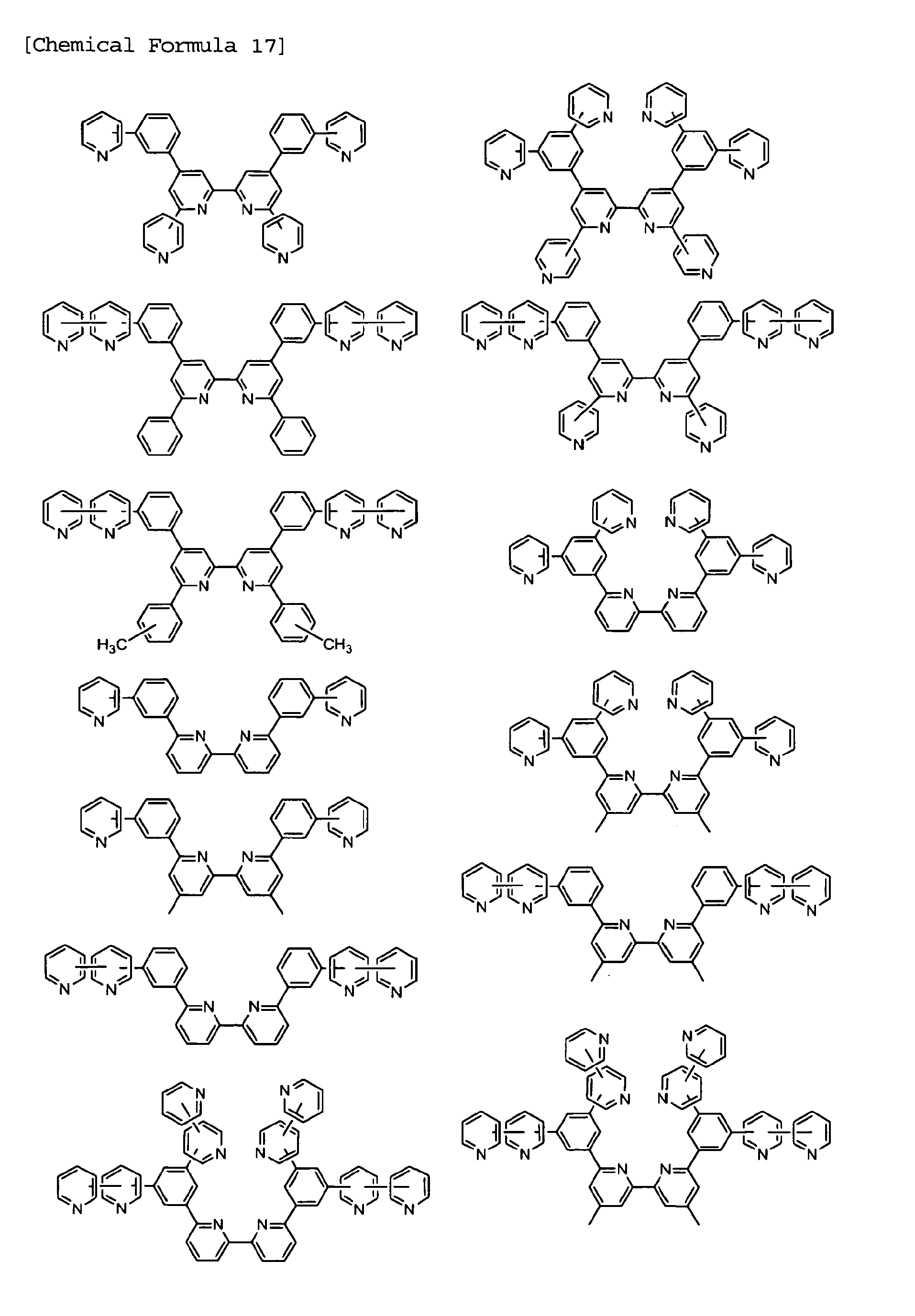

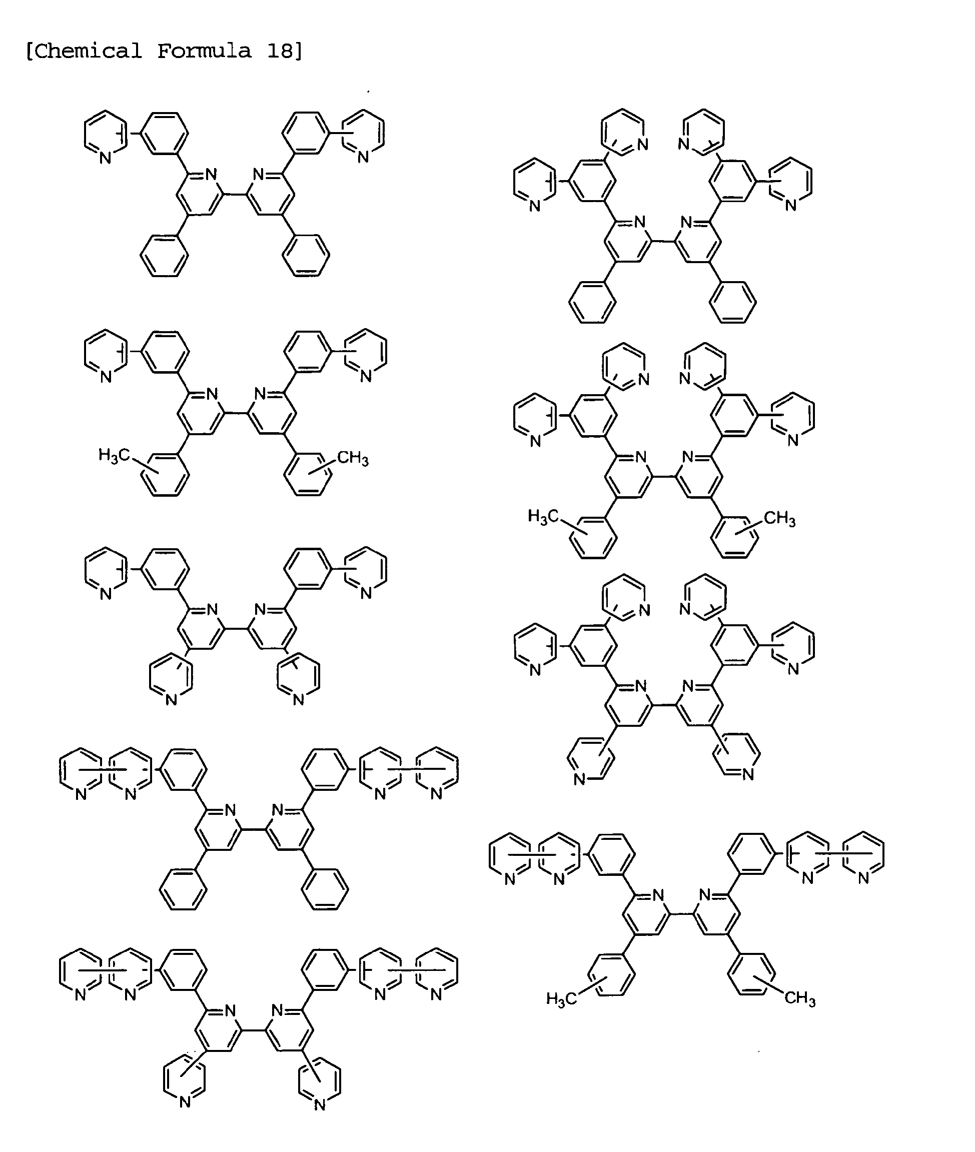

The structure of the general formula (4) is expressed by way of unit notation of Xc, Xd, Xe, and Xf, as shown below (Chemical Formula 19). Based on this notation, a particular example of a structure of bipyridine rings is shown in (Chemical Formula 20).

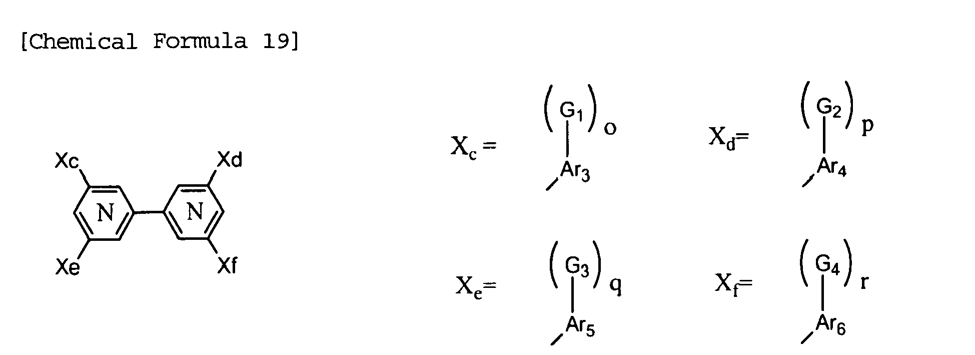

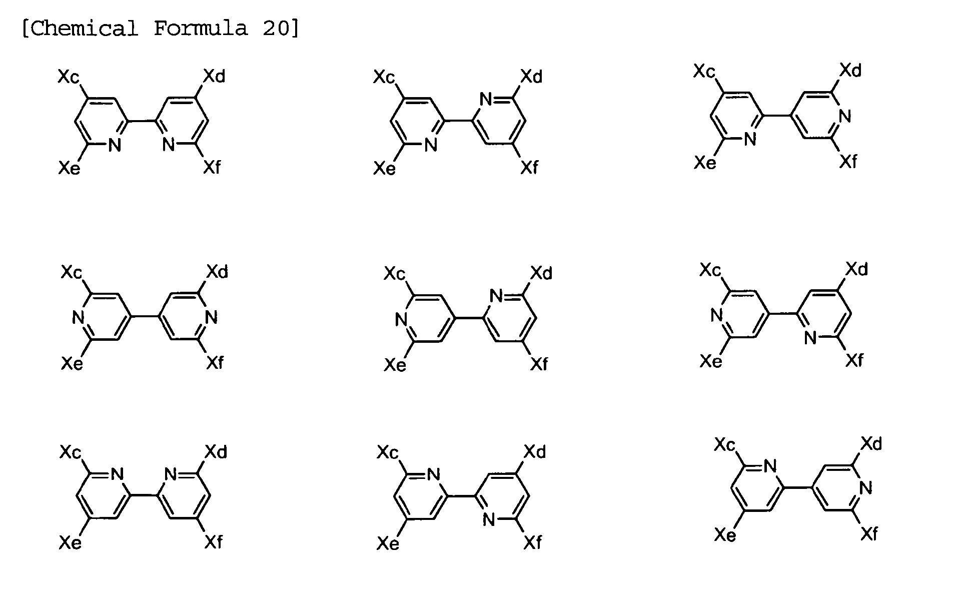

Further, a particular example of a substituent bonded with Xc, Xd, Xe, and Xf in the above-mentioned (Chemical Formula 19) is given below (Chemical Formula 21). In addition, X in (Chemical Formula 21) indicates a position bonded with a pyridine ring of (Chemical Formula 19).

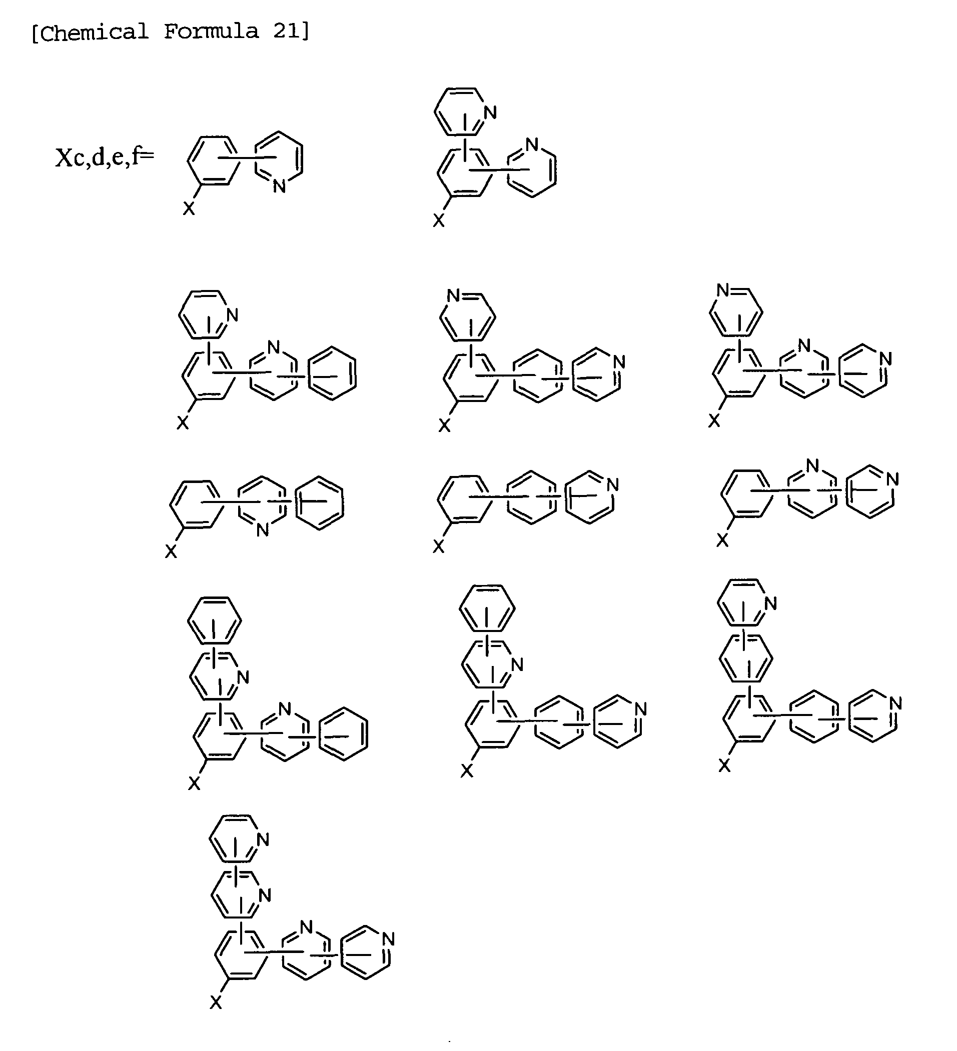

Of the bipyridine derivatives expressed by the above-mentioned general formulae (4) and (5), a structure of a particular compound is illustrated below.

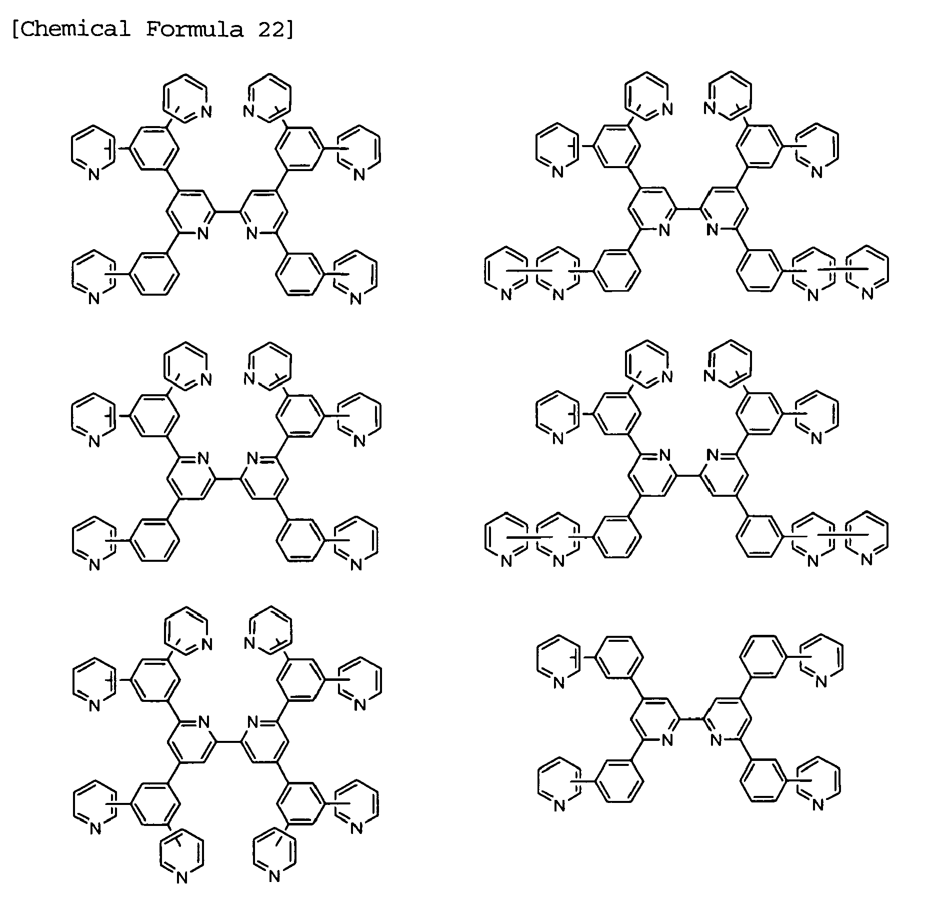

The structures of the general formulae (6) - (8) are expressed by way of unit notation of Xg and Xh, as shown below (Chemical Formula 23). Based on this notation, a particular example of a structure of bipyridine rings is shown in (Chemical Formula 24).

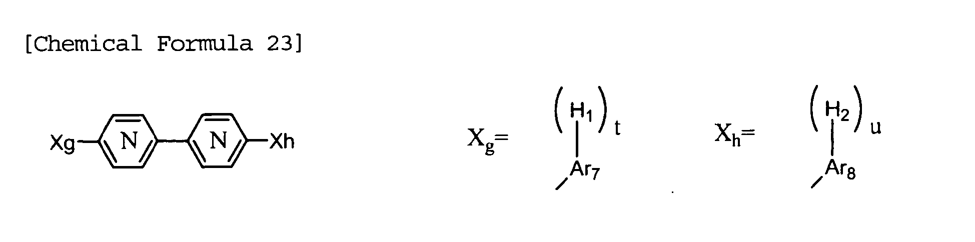

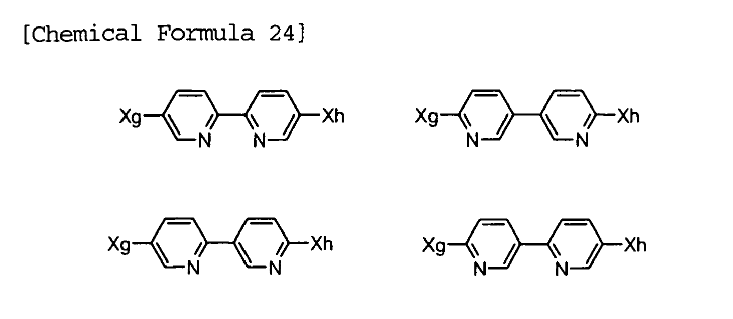

Further, a particular example of a substituent bonded with Xg and Xh in the above-mentioned (Chemical Formula 23) is given below (Chemical Formula 25). In addition, X in (Chemical Formula 25) indicates a position bonded with the pyridine ring of (Chemical Formula 23).

However, with respect to the bipyridine ring bonded at 2, 2' or 2, 3', it is assumed that Xg and Xh units consisting of aromatic members and Xg and Xh units containing a carbazolyl group are excluded.

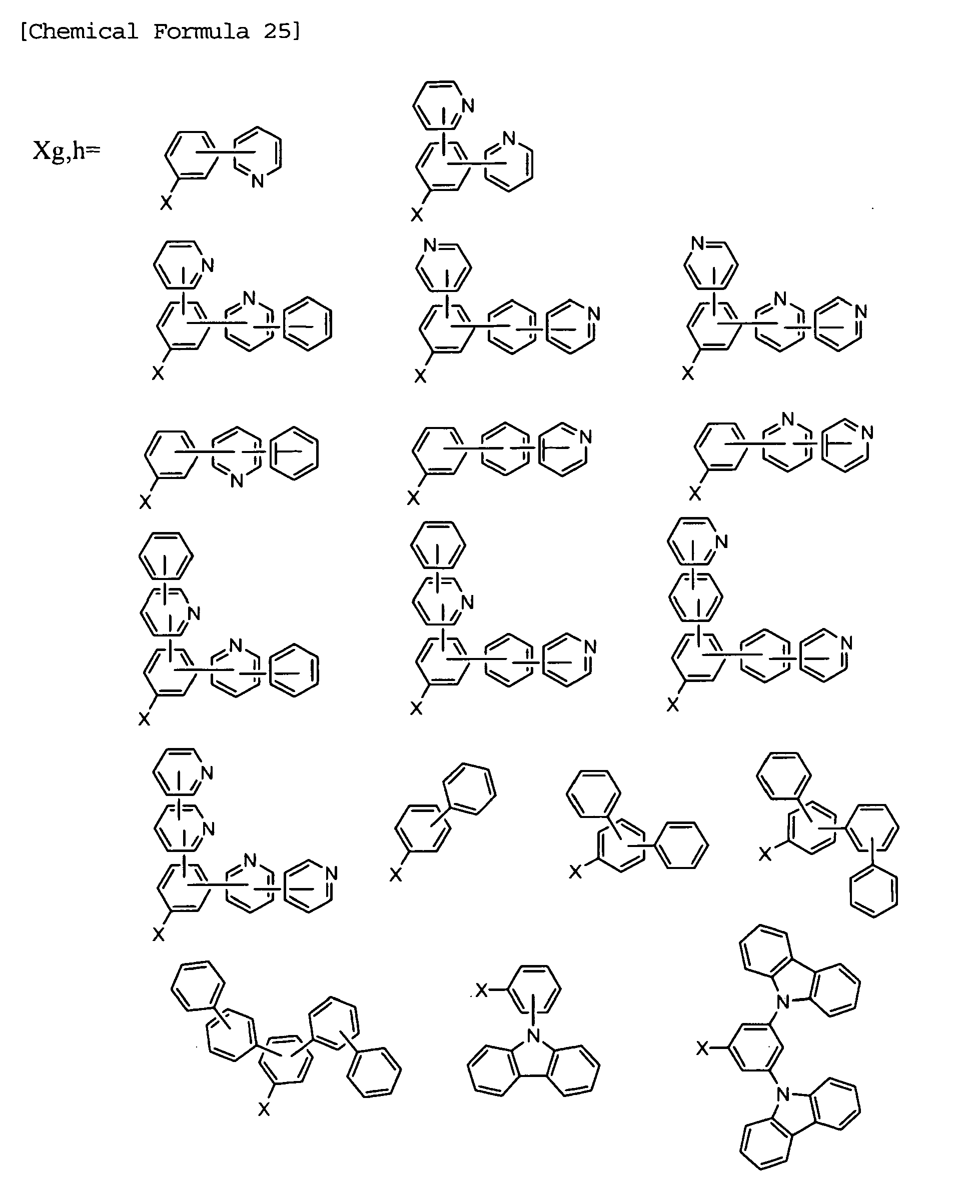

Of the bipyridine derivatives expressed by above-mentioned general formulae (6) - (8), a structure of a particular compound is illustrated below.

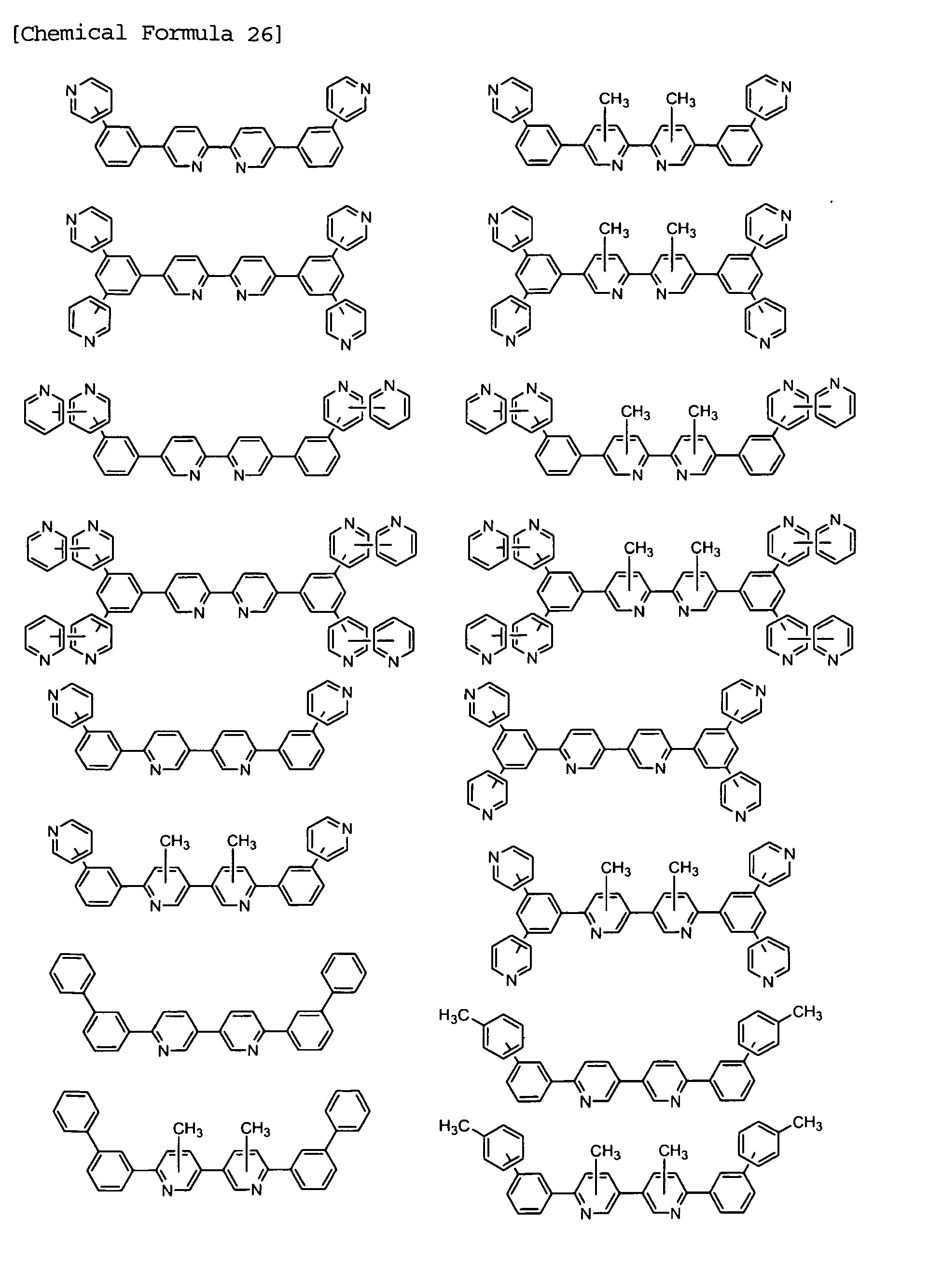

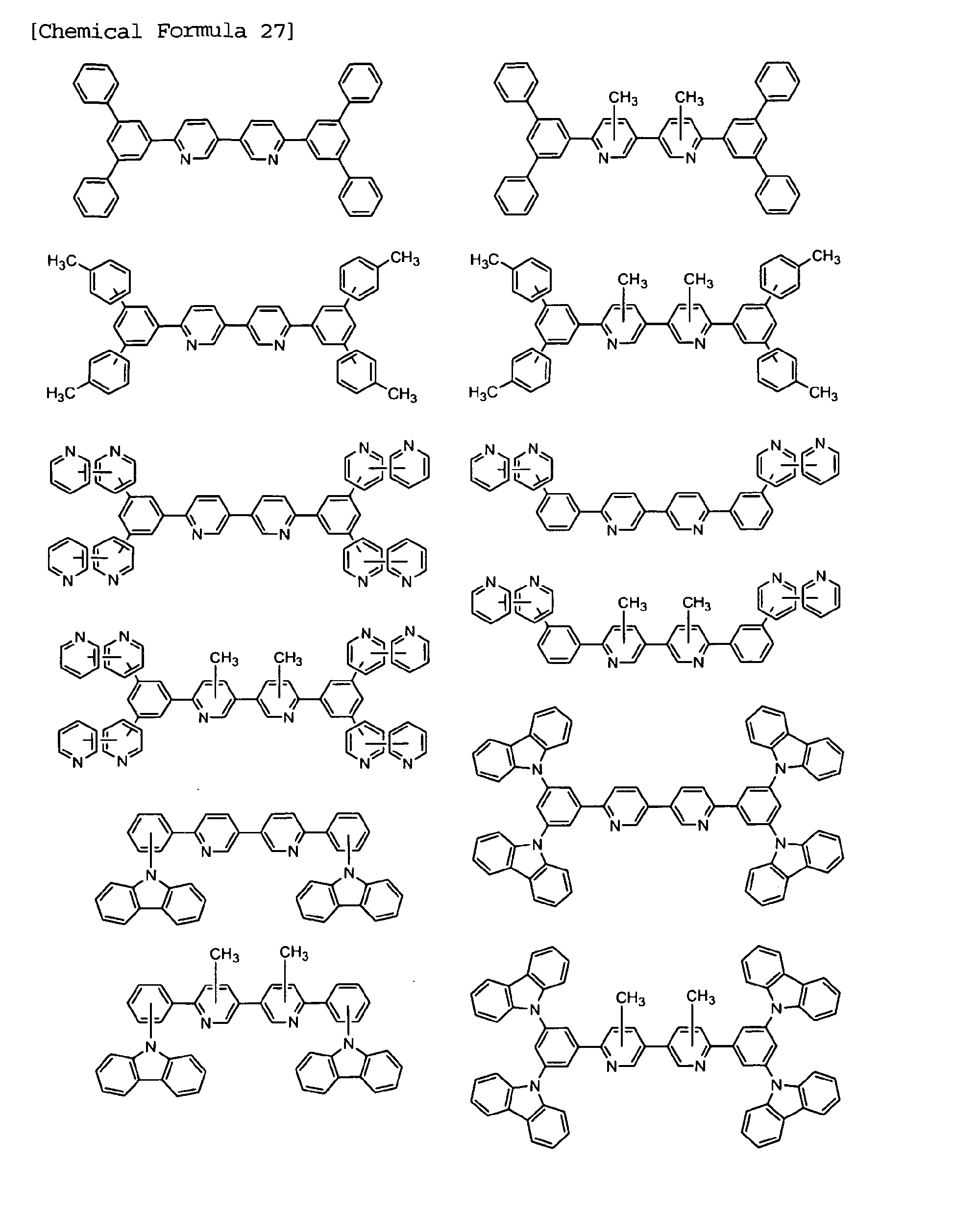

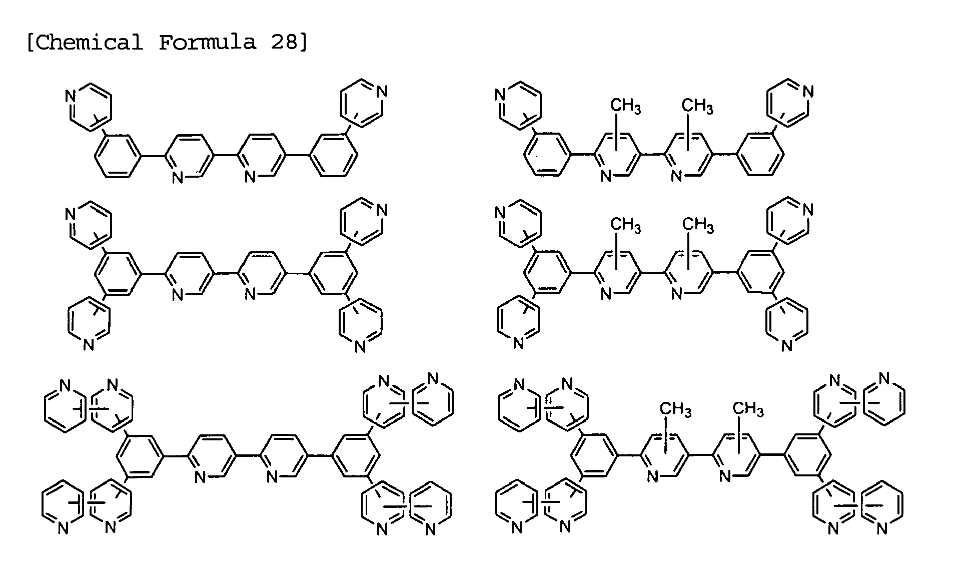

In particular, the above-mentioned bipyridine derivative can be manufactured by a method as shown below, but the manufacturing method is not limited thereto.

First, as a method of introducing the bipyridine rings, it is possible to employ methods using a (ring-closure reaction). Especially, the Krohnke method is preferred.

Further, it is also possible to carry out the introduction using a halogenated pyridine derivative. In particular, a halogenated pyridine derivative is reacted with distannane, diborane, etc., in the presence of a transition metal catalyst, such as palladium, nickel, etc., or the halogenated pyridine is reacted with organic lithium reagents, such as butyl lithium, and then reacted with chlorostannane, trialkoxyborane, a zinc halide, etc., whereby organic-metal reagents, such as an organotin reagent, an organoboron reagent, and an organozinc reagent, are obtained. Then, these are reacted and synthesized with a halogenated pyridine derivative by means of the transition metal catalyst, such as palladium, nickel, etc. Above all, a technique is particularly preferable in which the organotin reagent or the organoboron reagent is generated in the presence of a transition metal catalyst, to be reacted with the halogenated pyridine derivative.

As examples of other methods, there may be mentioned ones of using Raney nickel, palladium carbon, butyl lithium, boron trifluoride ether complex, etc., and dimerization through a sulfinyl pyridine derivative. In addition, there may also be mentioned a method of halogenating a bipyridine derivative directly and introducing the bipyridine main rings.

Further, as examples of the method of manufacturing a new bipyridine derivative in accordance with the present invention, there may be mentioned a method of forming a halogen-substituted precursor including the bipyridine main rings as described above to obtain the new bipyridine derivative, or a method of forming the bipyridine main rings later to obtain the new bipyridine derivative. In particular, the former method is preferred.

Hereafter, examples of synthesizing the halogen-substituted precursor and the new bipyridine derivative will be shown.

The precursor of the derivative expressed by the above-mentioned general formula (1) or (4) can be processed to synthesize the halogen-substituted precursor by way of the Krohnke method.

The precursor of the derivative expressed by the above-mentioned general formula (2), (3) or (5) can be synthesized by a similar method. For example, an acetyl derivative corresponding to a halogenated or unhalogenated aldehyde derivative is reacted in an acetic acid solution, an alcohol solvent, etc., alone or in a mixed solvent in the presence of an acid, or in an alcohol and/or water solvent in the presence of a base, such as sodium hydroxide etc., to obtain an intermediate (α, β-unsaturated carbonyl compound). Then, this intermediate and a corresponding halogenated or unhalogenated acylpyridinium salt are heated in a solvent, such as an acetic acid solution, methyl alcohol, and reacted with ammonium acetate, to thereby synthesize the precursor.

The precursor of the derivative expressed by the above-mentioned general formula (6) can be synthesized by reaction of a dihalogenated bipyridine derivative synthesized in advance with a corresponding organometallic reagent, such as an organoboron reagent, an organotin reagent, an organozinc reagent, etc., in the presence of a transition metal catalyst, for example. Further, employing the synthesized dihalogenated bipyridine derivative as the organometallic reagent, such as the organoboron reagent, the organotin reagent, the organozinc reagent, etc., then it is reacted with a corresponding halide to complete the synthesis. Alternatively, the intermediate is obtained by regioselective reaction of the dihalogenated pyridine derivative with a corresponding organometallic reagent, such as the organoboron reagent, the organotin reagent, the organozinc reagent, etc., in the presence of the transition metal catalyst, to thereby form the bipyridine main rings and complete the synthesis.

The precursor of the derivative expressed by general formula (7) can be obtained such that a dibromobipyridine derivative is synthesized by way of a known method (for example, J. Org. Chem., 67, p.443 (2002)), and this derivative and a halogenophenylboronic acid are reacted in the presence of a transition metal catalyst.

As examples of the transition metal catalyst in this case, there may be mentioned tetrakis(triphenylphosphine)palladium(0), PdCl₂[1,1'- bis(diphenylphosphino)ferrocene], PdCl₂[bis(triphenylphosphine)], etc., In particular, PdCl₂[bis(triphenylphosphine)] is preferred.

As examples of the solvent, there may be mentioned methanol, ethanol, butanol, methyl cellosolve, ethyl cellosolve, tetrahydrofuran, 1,4-dioxane, 1,2-dimethoxyethane, toluene, N,N-dimethylformamide, etc. Usually, it is often used by mixing with water and to dissolve the base, but it may also be used alone. Further, it is possible to use a mixed solvent of three types of solvents, such as water - ethanol - toluene. The water - ethanol - toluene solvent is preferred.

As the base, it is possible to use a carbonate, a hydrogen carbonate, a fluoride salt, a hydroxide, a phosphate of an alkaline metal, and a carbonate, a hydroxide, etc., of an alkaline earth metal. For example, there may be mentioned sodium carbonate, potassium carbonate, sodium hydrogen carbonate, potassium hydrogen carbonate, potassium fluoride, cesium fluoride, sodium hydroxide, potassium hydroxide, barium hydroxide, cesium carbonate, tripotassium phosphate, etc. Among these, sodium carbonate is particularly preferred.

The method of synthesizing the precursor of the derivative expressed by general formula (8) is carried out in such a way that 2,5-dibromopyridine, 2,5-dibromopicoline, etc., are used as materials and reacted with halogenophenylboronic acid in the presence of a transition metal catalyst, such as palladium, nickel, etc., to thereby introduce halogenophenyl group into a second position of these materials regioselectively, for example.

As examples of the transition metal catalyst in this case, there may be mentioned tetrakis(triphenylphosphine)palladium(0), PdCl₂[1,1'- bis(diphenylphosphino)ferrocene], PdCl₂[bis(triphenylphosphine)] , etc. In particular, tetrakis(triphenylphosphine)palladium(0) is preferred.

As for the solvent and base, ones similar to those in the case of synthesizing the precursor of the derivative expressed by the general formula (7) above can be used.

After introduction of the halogenophenyl group, the halogen group left in a fifth position is reacted with distannane, diborane, etc., in the presence of the transition metal catalyst, such as palladium, nickel, etc., or, a pyridine halide is reacted with an organolithium reagent, such as butyl lithium, then reacted with chlorostannane, trialkoxyborane, zinc halide, etc., to thereby obtain the organometallic reagents, such as an organotin reagent, an organoboron reagent, an organozinc reagent, etc. Further, these are reacted with the material having introduced the halogenophenyl group in the presence of the transition metal catalyst, such as palladium, nickel, etc., whereby the precursor can be synthesized. Especially, a method of generating an organotin reagent and an organoboron reagent in the presence of the transition metal catalyst so as to be reacted with the material having introduced the halogenophenyl group is preferred.

As for the transition metal catalyst in this case, it is possible to use the material similar to the above. In particular, PdCl₂[bis(triphenylphosphine)] is preferred.

As examples of the solvent, there may be mentioned tetrahydrofuran, 1,4-dioxane, 1,2-dimethoxyethane, toluene, N,N-dimethylformamide, dimethylsulfoxide, etc. In particular, dimethylsulfoxide is preferred.

As for the base, it is possible to use the material similar to the above. In particular, potassium carbonate is preferred.

Of the bipyridine derivatives in accordance with the present invention, what is expressed by the general formulae (1)-(8) can be prepared by coupling reaction of the precursor obtained by the above-mentioned method with a corresponding boronic acid derivative (Suzuki reaction), by coupling reaction with an organozinc reagent (Negishi reaction), by coupling reaction with an organotin reagent (Stille coupling) , etc.

Further, the precursor whose halogen group is suitably selected from fluorine, chlorine, bromine, and iodine is synthesized, allowing the coupling reaction to have selectivity, so as to prepare an asymmetrical bipyridine derivative.

Furthermore, the precursor having bipyridine main rings and synthesized by the above-mentioned method is reacted with distannane, diborane, etc. in the presence of the transition metal catalyst, such as palladium, nickel, etc., or a pyridine halide is reacted with organolithium reagent, such as butyl lithium etc., and then reacted with chlorostannane, trialkoxyborane, zinc halide, etc., to thereby obtain organometallic reagents, such as an organotin reagent, an organoboron reagent, an organozinc reagent , etc. Then, these are reacted with a corresponding halide in the presence of the transition metal catalyst, such as palladium, nickel, etc. , to thereby synthesize a new bipyridine derivative, also.

Hereafter, description will be carried out with reference to the general formula (1). As shown in the following (Chemical Formula 29), a method of coupling boronic acid derivative to a halide is preferred. In the following formula, A represents A₁ and A₂ in the general formula (1), and X represents a halogen group. 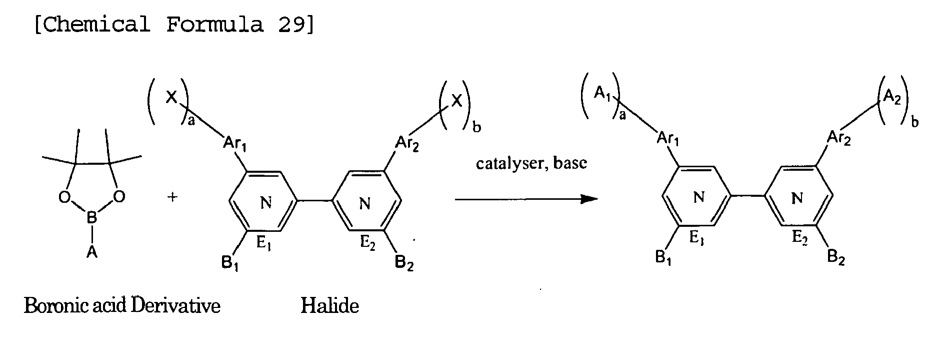 Further, the halide in the above formula is used as a boronic acid derivative, and subjected to coupling reaction with a corresponding halide, so that a new bipyridine compound can also be synthesized. In addition, this boronic acid derivative can be prepared from a halide etc. by way of a known manufacturing method (see Tetrahedron, 57, p.9813 (2001), J. Org. Chem., 60, p. 7508 (1995)).

The synthesis of the above-mentioned bipyridine derivative is carried out by coupling reaction using the transition metal catalyst in the presence of the base.

As examples of the base to be used, there may be mentioned a carbonate, a hydrogen carbonate, a fluoride salt, a hydroxide and a phosphate of an alkaline metal, as well as a carbonate and a hydroxide of an alkaline earth metal. More particularly, there may be mentioned sodium carbonate, potassium carbonate, sodium hydrogen carbonate, potassium hydrogen carbonate, potassium fluoride, cesium fluoride, sodium hydroxide, potassium hydroxide, barium hydroxide, cesium carbonate, tripotassium phosphate , etc. In particular, sodium carbonate and tripotassium phosphate are preferred.

Further, as for the transition metal complex to be used as a catalyst, it is possible to use one that is synthesized beforehand. Alternatively, the precursor compound and ligand of the transition metal complex may be used at the same time to generate it within a reaction system. As examples of the precursor compound of the transition metal complex, there may be mentioned palladium acetate, palladium chloride, tris(dibenzylideneacetone)dipalladium(0), palladium-carbon, etc. Among these, palladium acetate and tris(dibenzylideneacetone)dipalladium(0) are particularly preferred. Further, as examples of the ligand, there may be mentioned triphenylphosphine, tricyclohexylphosphine, tri(tert-butyl)phosphine, trimethyl phosphite, bisdiphenylphosphino ferrocene, etc. In particular, tricyclohexylphosphine and tri(tert-butyl)phosphine are preferred.

The synthesis of the above-mentioned bipyridine derivative is usually performed in an inert atmosphere, such as nitrogen and argon, which does not contain oxygen.

Further, as examples of the solvent to be used, there may be mentioned methanol, ethanol, butanol, methyl cellosolve, ethyl cellosolve, tetrahydrofuran, 1,4-dioxane, 1,2-dimethoxyethane, toluene, N,N-dimethylformamide , etc. Usually, it is often used by mixing with water to dissolve the base, but it may also be used alone. Further, it is possible to use a mixed solvent of three types of solvents, such as water - ethanol - toluene. Above all, a mixture solvent of 1,4-dioxane and water is preferred. Although solvent usage is 3-100 times, by weight, the halide usually, the usage of 10-80 times by weight is preferred.

Although a reaction temperature is within a range of 20-200°C, the range of 50-120°C is preferred. Reaction time is approximately 1-100 hours.

The bipyridine derivative synthesized as described above can be purified by column chromatography, recrystallization, or sublimation. Further, it is possible to combine any of these purification methods.

In the column chromatography, it can be refined by eluting it with a suitable solvent, using silica gel, alumina, florisil, etc. as a filler.

Further, as examples of the solvent used for recrystallization purification, there may be mentioned N-methylpyrrolidone, N,N-dimethylformamide, N,N-dimethylacetamide, dimethylsulfoxide, chlorobenzene, o-dichlorobenzene , etc.

Furthermore, as for the synthesis of the bipyridine derivative expressed by the above-mentioned general formula (8), it is possible to employ a method similar to the above when introducing the above-mentioned bipyridine derivative halogen-substituted precursor, and the aromatic-hydrocarbon group represented by phenyl group, and π electron-deficient aromatic heterocyclic group represented by pyridyl group.

Still further, also in the reaction with an electron donating heterocyclic group, such as a carbazolyl group, a similar method using the transition metal catalyst as described above can be employed.

As for the transition metal complex used as the catalyst, it is possible to use one that is synthesized beforehand. Alternatively, the precursor compound and ligand of the transition metal complex may be used at the same time to generate it within a reaction system. As examples of the precursor compound of the transition metal complex, there may be mentioned palladium acetate, palladium chloride, tris(dibenzylideneacetone)dipalladium(0), etc. Among these, palladium acetate and tris(dibenzylideneacetone)dipalladium(0) are particularly preferred. Further, as examples of the ligand, there may be mentioned tricyclohexylphosphine, tri(tert-butyl)phosphine, bisdiphenylphosphino ferrocene, etc. Among these, bisdiphenylphosphino ferrocene and tri(tert-butyl)phosphine are particularly preferred. As examples of the base used, there may be mentioned a carbonate, a hydrogen carbonate, a fluoride salt, a hydroxide and a phosphate of an alkaline metal, and a carbonate, a hydroxide, an alcoholate of an alkaline earth metal. In particular, there may be mentioned sodium carbonate, potassium carbonate, sodium hydrogen carbonate, potassium hydrogen carbonate, potassium fluoride, cesium fluoride, sodium hydroxide, potassium hydroxide, barium hydroxide, cesium carbonate, tripotassium phosphate, t-BuoNa , etc. Among these, potassium carbonate and cesium carbonate are particularly preferred. As examples of the solvent, there may be mentioned tetrahydrofuran, 1,4-dioxane, toluene, xylene, N,N-dimethylformamide, etc. Among these, xylene and toluene are particularly preferred.

The bipyridine derivative as described above in accordance with the present invention can be used as an organic EL material, in particular as an electron injection material and an electron transport material. It can be suitably applied to the electron injection layer, the electron transport layer, etc., in an organic EL element. In this case, it can also be used by combining it with other electron transport material.

Further, it can also be used by combining it with a suitable light-emitting material (dopant).

Furthermore, the organic EL element in accordance with the present invention is an organic EL element which is provided, on a substrate, with a pair of electrodes (an anode and a cathode) and at least one organic layer between the above-mentioned anode and cathode, and at least one layer of the above-mentioned organic layers contains therein the above-mentioned bipyridine derivative.

As for the above-mentioned organic EL element, it is preferable that a hole transport layer and a hole injection layer are arranged between the above-mentioned anode and light emitting layer in terms of improvement in luminous efficiency. Further, it is preferable that the electron transport layer, the electron injection layer and a hole inhibition layer are arranged between the above-mentioned cathode and light emitting layer.

As particular example of these layer structures, there may be mentioned "anode / light emitting layer / cathode", "anode / hole transport layer / light emitting layer / electron transport layer / cathode", "anode / hole injection layer / hole transport layer / light emitting layer/ electron transport layer / electron injection layer / cathode", "anode / hole injection layer / hole transport layer / light emitting layer / hole inhibition layer / electron transport layer / electron injection layer / cathode" etc. In addition, each layer can also be of a single layer or of a plurality of layers.

Furthermore, it is also possible to employ a known layer structure further including a hole transport light emitting layer, an electron transport light-emitting layer, etc.

The materials used for the above-mentioned hole injection layer , hole transport layer, and hole transport light emitting layer is not particularly limited, but can be used by suitably selecting from conventional ones.

In particular, there may be mentioned arylamine derivatives, such as bis(di(p-tolyl)aminophenyl)-1,1-cyclohexane (commonly known as TAPc), Spiro-TPD (Chemical Formula 30), N,N'-diphenyl-N,N'-bis(3-methyl phenyl)-1,1'-biphenyl-4,4'-diamine (commonly known as TPD), N,N'-diphenyl-N,N'-bis(1-naphthyl)-1,1'-biphenyl-4,4'-diamine (commonly known as α-NPD), TPTE (Chemical Formula 31), starburst amine (Chemical Formula 32), styryl amine (Chemical Formula 33), TCTA (Chemical Formula 34), 3DTAPBP (Chemical Formula 35) , etc.

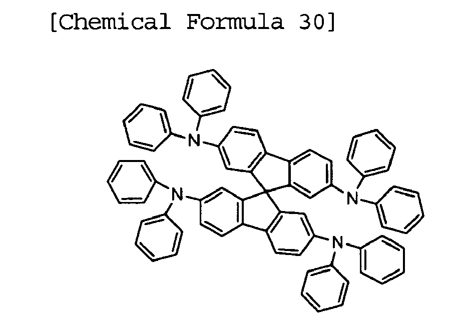

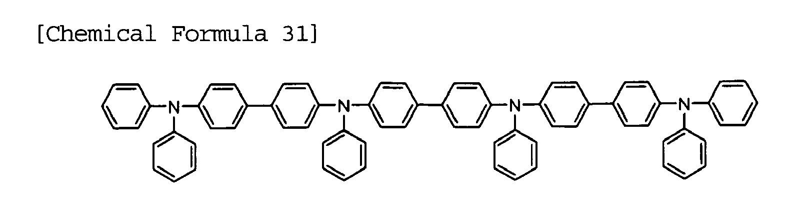

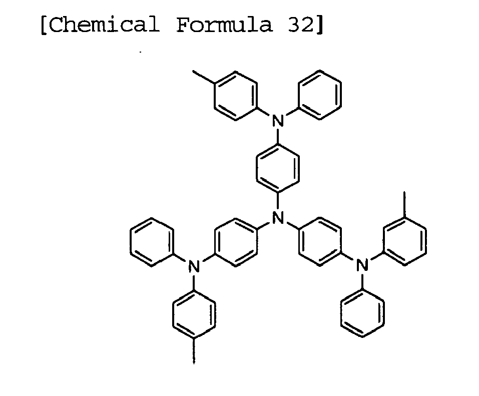

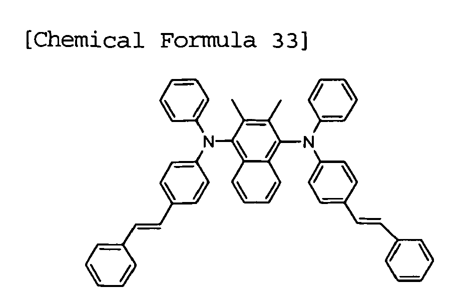

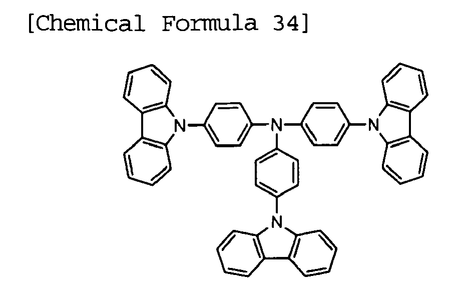

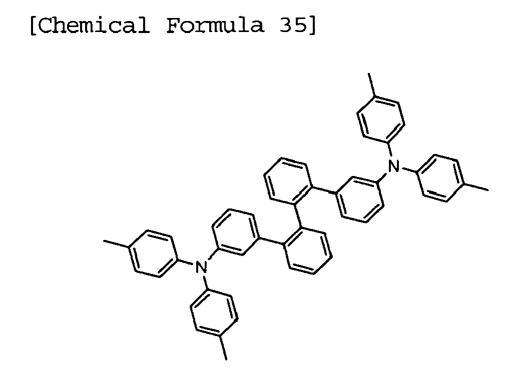

Further, it is possible to use carbazole compounds as shown in the following (Chemical Formula 36) and its derivative, a pyrazoline derivative, a styrylaryl derivative, distyrylaryl compounds as shown in the following (Chemical Formula 37) and its derivative.

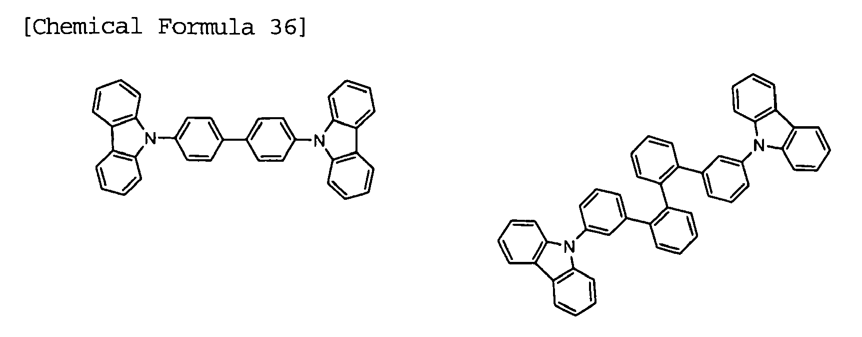

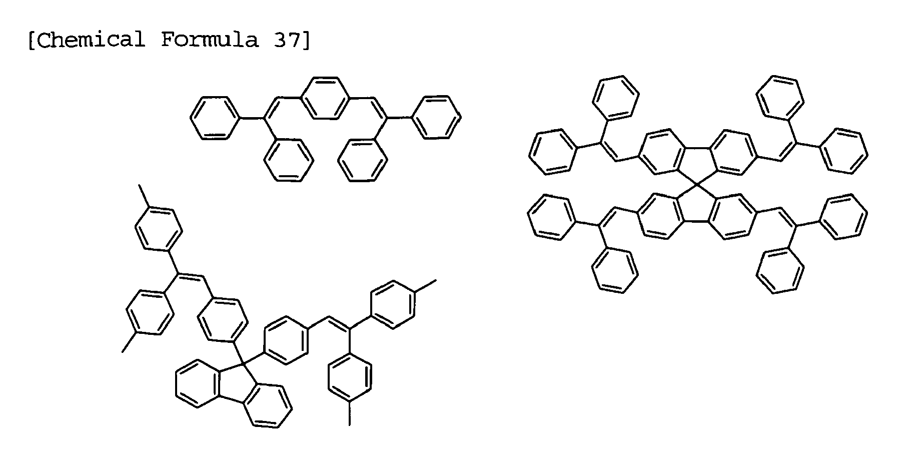

Alternatively, it is also possible to use fused polycyclic aromatic hydrocarbon compounds, such as anthracene, triphenylene, perylene, naphthalene, pyrene, coronene, chrysene, naphthacene, tetracene, phenanthrene, etc., and their derivatives, as well as polycyclic compounds, such as paraterphenyl, quaterphenyl, m-phenylene (Chemical Formula 38) , etc., and their derivatives.

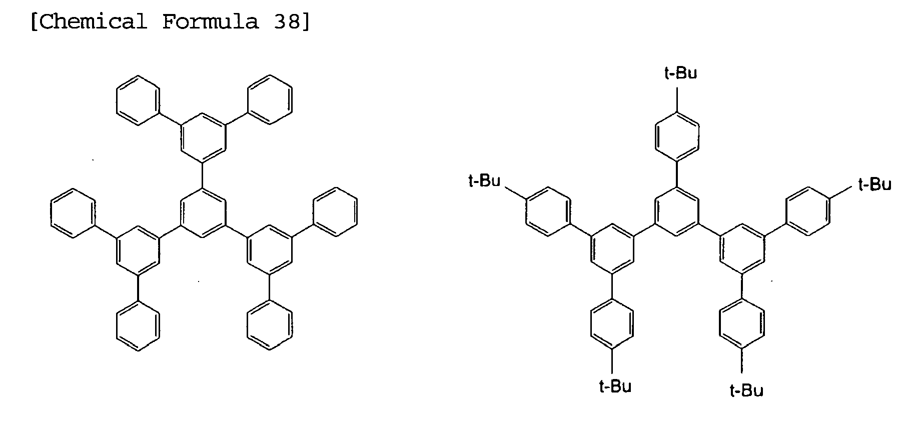

Furthermore, the hole injection layer, the hole transport layer, and the hole transport light emitting layer may employ the above-mentioned organic compound which is dispersed in a polymer, an oligomer, or a dendrimer, or which is polymerized, oligomerized, or dendrimerized.

Still further, it is also possible to use so-called π conjugated polymers, such as polyparaphenylenevinylene, polyfluorene, its derivative, etc., a hole transport unconjugated polymer represented by poly(N-vinylcarbazole), and σ conjugated polymers etc. represented by polysilanes. Yet further, it is also possible to use so-called conjugated oligomers, such as a fluorene oligomer, its derivative, etc.

Further, besides the above-mentioned materials, the hole injection layer may employ conductive polymers, such as metal phthalocyanines, non-metal phthalocyanines, carbon film, fluorocarbon film, polystyrenesulfonic acid (PEDOT-PSS), polyaniline, etc.

Furthermore, the above-mentioned organic compound may be reacted with organic oxidizing dopants, such as tetracyanoquinodimethane, trinitrofluorenone, etc., and inorganic oxidizing dopants, such as vanadium oxide, molybdenum oxide, tungstic oxide, aluminum oxide, etc., to form a radical cation, and can be used as the hole injection transport layer.

The oxidizing dopant concentration in the hole injection transport layer is not particularly limited, but it is preferable to be approximately 0.1 - 99 % by weight.

Further, the material used for the electron injection layer, the electron transport layer, the electron transport light-emitting layer, the hole inhibition layer is not particularly limited, but can be suitably used by selecting from conventionally known ones.

In particular, there may be mentioned polycyclic compounds, such as paraterphenyl, quaterphenyl, m-phenylene (Chemical Formula 38), etc., and their derivatives, as well as a styrylaryl derivative, distyrylaryl compounds (Chemical Formula 37), and its derivative.

Further, it is possible to use fused polycyclic aromatic hydrocarbon compounds, such as anthracene, triphenylene, perylene, naphthalene, pyrene, coronene, chrysene, naphthacene, tetracene, phenanthrene, etc., and their derivatives as well as heterocyclic compounds, such as phenanthroline, bathophenanthroline, bathocuproine, phenanthridine, acridine, quinoline, quinoxaline, pyridine (Chemical Formula 39), pyrimidine, pyrrole, pyrazole, pyridazine, pyrazine, phthalazine, naphthyridine, quinazoline, cinnoline, thiazole, oxadiazole, oxazole, triazine, phenazine, imidazole, benzoxazole, benzothiazole, benzimidazole, triazole, porphyrin, etc., and their derivatives.

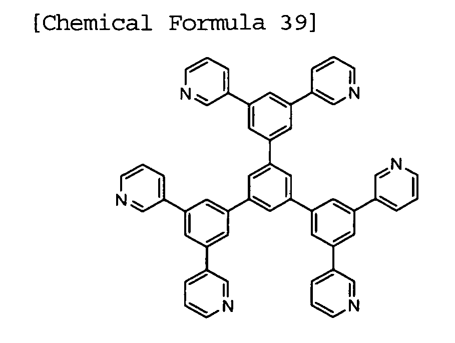

Further, it is also possible to use metal complex materials, for example, a quinolinol aluminum complex, a benzoxazole zinc complex, a benzothiazole zinc complex, an azomethine zinc complex, a europium complex, an iridium complex, a platinum complex, etc., in which Al, Zn, Be, Ir, Pt, Tb, Eu, etc. are provided as central metals, and oxadiazole, thiadiazole, phenylpyridine, and quinoline structures are provided as ligands.

It is also possible to use organosilicon compounds, such as silole, siloxane, etc., and their derivatives, organoboron compounds, such as triarylborane etc., and their derivatives, and pentavalent phosphorus compounds, such as triarylphosphine oxide etc., and their derivatives etc. Pentavalent sulfur compounds, such as diarylsulfoxide, its derivative, etc. can also be used.

Furthermore, the hole injection layer, the hole transport layer, and the hole transport light emitting layer may employ the above-mentioned organic compound which is dispersed in a polymer, an oligomer, or a dendrimer, or which is polymerized, oligomerized, or dendrimerized.

Further, it is possible to use the so-called π conjugated polymers, such as polyparaphenylenevinylene, polyfluorene, its derivative, etc. as well as an electron transport unconjugated polymer etc. represented by polyvinyloxadiazole. Furthermore, it is possible to use the so-called conjugated oligomers, such as a fluoreneoligomer, its derivative, etc.

Further, besides the above-mentioned organic compounds, as for the electron injection layer, it is possible to use single metals, such as Ba, Ca, Li, Cs, Mg, Sr, W, etc., metal fluorides, such as magnesium fluoride, calcium fluoride, strontium fluoride, barium fluoride, lithium fluoride, cesium fluoride, etc., metal alloys, such as aluminum lithium alloy, etc., metal oxides, such as magnesium oxide, strontium oxide, aluminum oxide, etc., and organometallic complexes, such as sodium polymethylmethacrylate polystyrene sulfonate etc.

Further, single metals, such as Ba, Ca, Li, Cs, Mg, Sr, W, etc., metal oxides, such as magnesium oxide, strontium oxide, aluminum oxide, etc., metal salts, such as magnesium fluoride, calcium fluoride, strontium fluoride, barium fluoride, lithium fluoride, cesium fluoride, cesium chloride, strontium chloride, etc., and inorganic reducing dopants may be mixed or dispersed to form a radical anion, which can be used for the electron injection transport layer.

Furthermore, the above-mentioned organic compound may be reacted with an organic reducing dopant, such as 8-hydroxyquinoline type Cs, Li organometallic complex, etc., to form a radical anion, which can be used as the electron injection transport layer.

Although the reducing dopant concentration in the above-mentioned electron injection transport layers is not particularly limited, it is preferable to be approximately 0.1 - 99 % by weight.

Further, it is possible to constitute the light emitting layer of the organic EL element in accordance with the present invention by using a bipolar material. By bipolar material is meant a material which can convey both the hole and the electron and may emit light by itself.

The material used for a bipolar light emitting layer is not particularly limited. For example, there may be mentioned styrylaryl derivative, distyrylaryl compound (Chemical Formula 37) and its derivative, polycyclic aromatic compounds, such as paraterphenyl, quaterphenyl, m-phenylene (Chemical Formula 38), etc., and their derivatives, fused polycyclic aromatic hydrocarbon compounds, such as anthracene, triphenylene, perylene, naphthalene (Chemical Formula 40), pyrene, coronene, chrysene, naphthacene, tetracene, phenanthrene, etc., and their derivatives, carbazole compounds (Chemical Formula 36, Chemical Formula 41) and their derivatives, heterocyclic compounds, such as thiophene etc.

Further, as examples other than these derivatives, etc., there may be mentioned 4,4'-bis(2,2- diphenyl-ethene-1-yl)diphenyl (DPVBi) (Chemical Formula 42), spiro6 (Chemical Formula 43), 2,2',7,7'-tetrakis(carbazole-9-yl)-9,9'-spiro-bifluorene (Chemical Formula 44), 4,4'-di(N-carbazolyl)-2',3',5',6'-tetraphenyl-p-terphenyl (Chemical Formula 44), 1,3-bis(carbazole)-9-yl)-benzene (Chemical Formula 46), 3-tert-butyl-9,10-di(naphtha-2-yl)anthracene (commonly known as TBADN) (Chemical Formula 47).

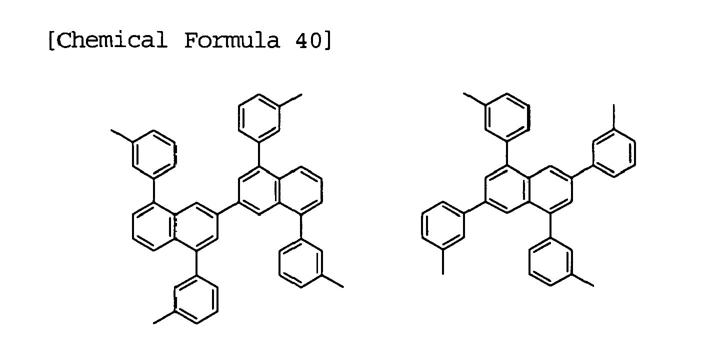

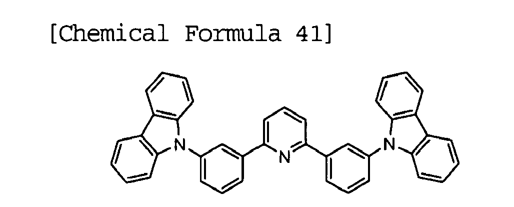

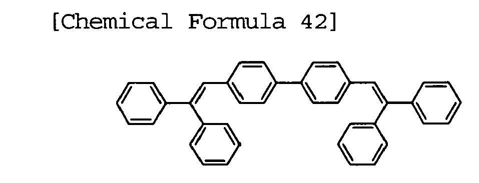

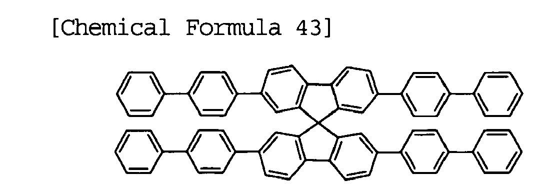

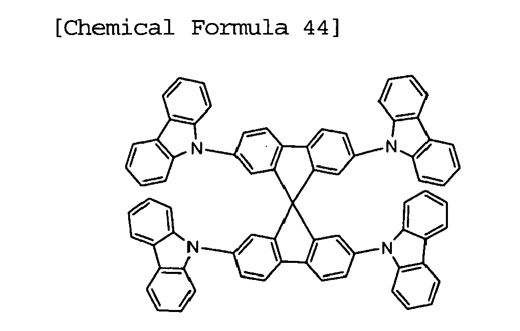

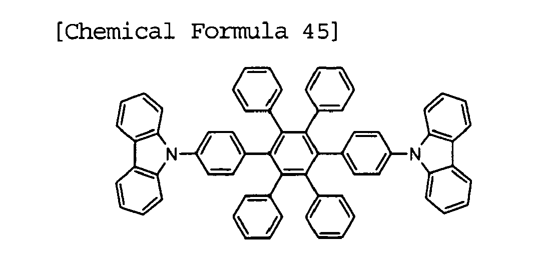

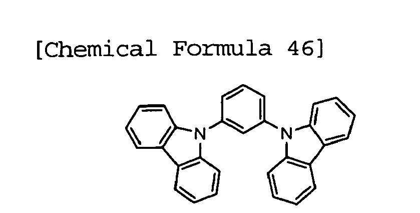

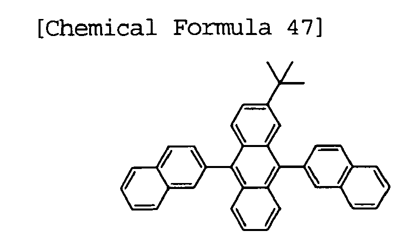

Further, the bipolar material may employ the above-mentioned organic compound which is dispersed in a polymer, an oligomer, or a dendrimer, or which is polymerized, oligomerized, or dendrimerized.

Furthermore, it is also possible to use the so-called π conjugated polymers, such as polyparaphenylenevinylene, polyfluorene, and their derivatives, etc., and unconjugated polymers etc. represented by polyvinylcarbazole. Yet further, it is also possible to use the so-called conjugated oligomers, such as a fluoreneoligomer and its derivatives.

Still further, copolymers, such as poly(vinyltriarylaminevinyloxadiazole) etc., where monomers having a hole transport function and an electron transport function exist in the same molecule, and a dendrimer can also be used.

In the case where the above-mentioned hole transport light-emitting material, the electron transport light-emitting material, and the bipolar material are used as a host material of the light emitting layer, a guest material of the above-mentioned light emitting layer may be a fluorescence or phosphorescence material.

As examples, there may be mentioned polycyclic compounds, such as paraterphenyl, quaterphenyl, etc., and their derivatives as well as fused polycyclic aromatic hydrocarbon compounds, such as a styrylaryl derivative, a distyrylaryl compound, a tetraphenylbutadiene derivative, a pyrazoline derivative, an oxadiazole derivative, a coumarin derivative, a styrylamine (Chemical Formula 33) derivative, anthracene (Chemical Formula 48), triphenylene, perylene, naphthalene (Chemical Formula 49), pyrene, coronene, chrysene, naphthacene, tetracene, phenanthrene, etc. and their derivatives.

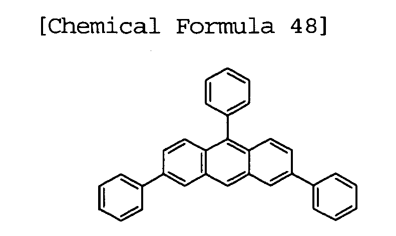

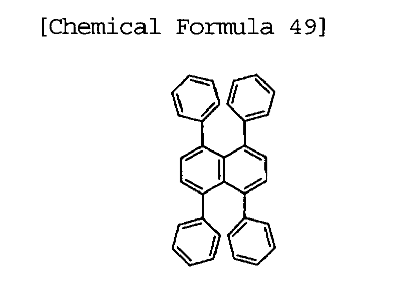

Further, there may be mentioned a quinacridone derivative, a squarylium derivative, a porphyrin derivative, a coumarin derivative, a dicyanopyran derivative, arylamine compounds, such as anthracenediamine, and their derivatives, fused polycyclic aromatic hydrocarbon compounds, such as perylene, rubrene, tetracene, decacyclene, and their derivatives, phenoxazone, a quinoxaline derivative, a carbazole derivative, a fluorene derivative.

Further, it is also possible to use metal complex materials, for example, a quinolinol aluminum complex, a benzoxazole zinc complex, a benzothiazole zinc complex, an azomethine zinc complex, a europium complex, a terbium complex, an iridium complex, a platinum complex, etc., in which Al, Zn, Be, Ir, Pt, Tb, Eu, etc. are provided as central metals, and oxadiazole, thiadiazole, phenylpyridine, and quinoline structures are provided as ligands. As particular examples, there may be mentioned Firpic (Chemical Formula 50), PQ₂Ir(dpm) (Chemical Formula 51), Irpiq₃ (Chemical Formula 52), Irppy₃ (Chemical Formula 53) complex and their derivatives.

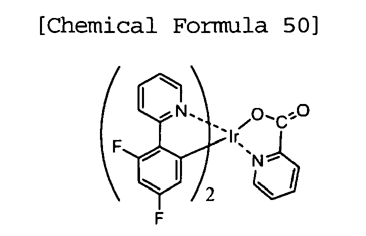

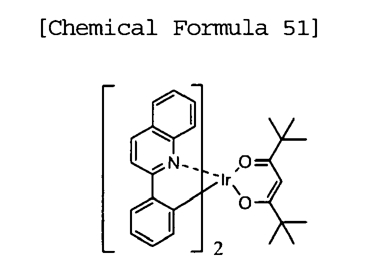

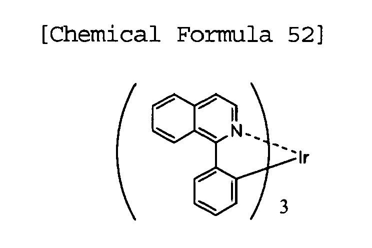

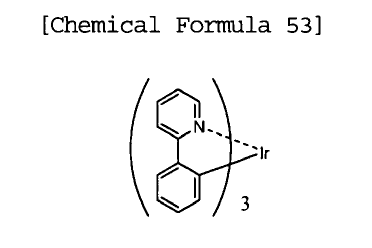

The formation of each of the above-mentioned layers can be performed by way of dry processes, such as a vacuum deposition process, a sputtering process, etc., and wet processes, such as an ink-jet process, a casting process, a dip coat process, a bar coat process, a blade coat process, a roll coat process, a photogravure coat process, a flexographic printing process, a spray coat process, etc. Preferably, the film formation is carried out by vacuum deposition.

Further, although a film thickness of each of the above-mentioned layers is suitably determined depending on its conditions in view of adaptability between the respective layers, the whole layer thickness to be required, etc., it is usually preferable to be within a range from 5 nm to 5 µm.

As for the electrode of the organic EL element in accordance with the present invention, it is preferable that a transparent electroconductive thin-film is formed on a transparent substrate.

The above-mentioned substrate serves as a support of an organic electroluminescence element. In the case where the substrate side is a light emitting side, it is preferable to use the transparent substrate which is transmissive in visible light. It is preferable that the optical transmissivity is 80% or more. More preferably, it is 85% or more. Most preferably, it is 90% or more.

In general, the above-mentioned transparent substrate employs glass substrates made of, such as for example, optical glass (BK7, BaK1, F2, etc.), silica glass, non-alkali glass, borosilicate glass, aluminosilicate glass, etc., and polymer substrates made of, such as for example, acrylic resins (PMMA, etc.), polycarbonate, polyether sulphonate, polystyrene, polyolefin, an epoxy resin, and polyester, such as polyethylene terephthalate etc.

Although the above-mentioned substrate having a thickness of approximately 0.1-10 mm is usually used, it is preferable that the thickness is 0.3-5 mm in view of mechanical strength, weight, etc. More preferably it is 0.5-2 mm.

Usually, the anode is provided on the above-mentioned substrate. Although this anode is made of a metal, an alloy, an electroconductive compound, etc., with a large work function (4 eV or more), it is preferable to be formed as a transparent electrode on the above-mentioned transparent substrate.

In general, metal oxides, such as indium tin oxide (ITO), indium zinc oxide, and zinc oxide, etc., are used for this transparent electrode. In particular, ITO is preferably used in terms of its transparency, conductivity, etc.

In order to secure the transparency and conductivity, it is preferable that a film thickness of this transparent electrode is 80-400 nm. More preferably, it is 100-200 nm.

It is preferable that the anode is usually formed by way of a sputtering process, a vacuum deposition process, etc., and formed as the transparent electroconductive thin-film.

On the other hand, the cathode which faces the above-mentioned anode is made of a metal, an alloy, and a conductive compound having a small work function (4 eV or less). As examples thereof, there may be mentioned aluminum, an aluminum-lithium alloy, a magnesium-silver alloy, lithium fluoride, etc. It can be of a single layer, or of plural layers in which the materials having different work function are combined.

It is preferable that a film thickness of the above-mentioned cathode is 10-500 nm. More preferably, it is 50-200 nm.

The above-mentioned cathode can be formed by forming a film by way of processes usually used, such as a sputtering process, an ion plating process, a vapor-deposition process, etc.

Hereafter, the present invention will be described more particularly with reference to Examples, but the present invention is not limited to the following Examples.

In Examples 1 and 2, bipyridine derivatives expressed by the general formula as shown in the following (Chemical Formula 54) were synthesized.

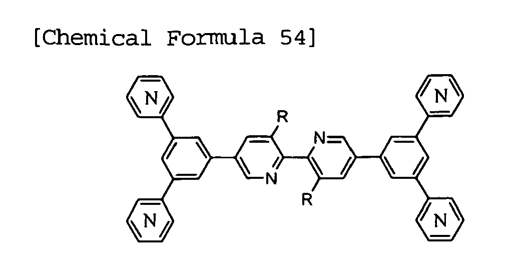

Of the bipyridine derivatives as shown in (Chemical Formula 54), one in which R=H and four terminal pyridyl groups are 4-pyridyl groups is abbreviated and referred to as "BP-1p", one in which R=H and four terminal pyridyl groups are 3-pyridyl groups is abbreviated and referred to as "BP-1m", one in which R=Me(methyl group) and four terminal pyridyl groups are 4-pyridyl groups is abbreviated and referred to as "BP-3p", and one in which R=Me(methyl group) and four terminal pyridyl groups are 3-pyridyl groups is abbreviated and referred to as "BP-3m", hereafter.

### [Example 1] (synthesis of BP-1 series)

BP-1p and BP-1m were respectively synthesized by a synthesis scheme shown below.

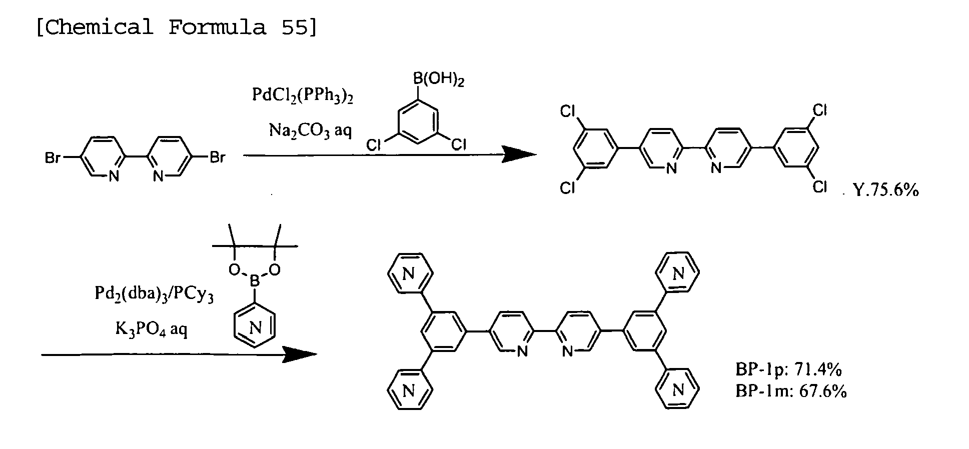

### (First Process: Synthesis of 5,5'-bis-(3,5-dichlorophenyl) -[2, 2'] bipyridinyl)

9.4 g (29.95 mmol) of 5,5'-dibromo2,2'-bipyridine synthesized by a known method (see J. Org. Chem., 67, p.443 (2002)), 20 g (29.95 mmol) of 3,5-dichlorophenylboric acid, 376 ml of toluene, 188 ml of ethanol, 66 ml of 2M sodium carbonate aqueous solution, and 2.1 g (2.995 mmol) of Pd(PPh₃)₂Cl₂ were prepared and reacted in a nitrogen atmosphere for 2 hours.

The precipitated crystal was filtered and washed with water and methanol. The filtrate was separated and a toluene layer was fractioned. The toluene layer was washed with water twice. After washing, a crude material obtained by collecting toluene was mixed with the filtered precipitate crystal. This crude material was subjected to a silica gel process, and the resulting crude material was recrystalized from dimethylacetamide and purified.

Structure identification was performed by way of mass spectrometry (MS) and ¹H-NMR, and it was confirmed that it was a target. Its yield was 10.1 g (75.6% yields).

### (Second Process: Synthesis of BP-1p)

2.0 g (4.483 mmol) of 5,5'-bis-(3,5-dichlorophenyl) -[2, 2'] bipyridinyl synthesized by the above-mentioned first process, 6.4 g (31.38 mmol) of 4-(4,4,5,5-tetramethyl-[1, 3, 2]dioxaborolane-2-yl)-pyridine, 96 ml of 1,4-dioxane, 32ml of 1.35M potassium phosphate aqueous solution, 0.4 g (0.4483 mmol) of Pd₂dba₃, and 0.31 g (0.8966 mmol) of PCy₃ were placed and reacted in a nitrogen atmosphere for 30 hours.

The precipitated crystal was filtered, and washed with water and methanol. After drying, a crude material was purified with a silica gel column using a developing solvent of chloroform-methanol mixture solvent.

Structure identification was performed by way of MS and ¹H-NMR, and it was confirmed that it was a target. Its yield was 2.0g (71.4% yields).

Further, this was sublimed and purified, then subjected to differential scanning calorimetry (DSC), and its melting point was 433.89°C.

### (Second Process: Synthesis of BP-1m)

3.0 g (6.724 mmol) of 5,5'-bis-(3,5-dichlorophenyl) -[2, 2'] bipyridinyl synthesized by the above-mentioned first process, 9.7 g (47.07 mmol) of 3-(4,4,5,5-tetramethyl-[1, 3, 2]dioxaborolane-2-yl)-pyridine, 144 ml of 1,4-dioxane, 47 ml of 1.35M potassium phosphate aqueous solution, 0.62 g (0.6724 mmol) of Pd₂dba₃, and 0.47 g (1.345 mmol) of PCy₃ were placed and reacted in a nitrogen atmosphere for 30 hours.

The precipitated crystal was filtered, and washed with water and methanol. After drying, its crude material was purified with a silica gel column using a developing solvent of chloroform-methanol mixture solvent.

Structure identification was performed by way of MS and ¹H-NMR, and it was confirmed that it was a target. Its yield was 2.8 g (67.6% yields).

Further, this was sublimed and purified, and its melting point (DSC) was 384.75 °C.

### [Example 2] (Synthesis of BP-3 series)

BP-3p and BP-3m were respectively synthesized by a synthesis scheme as shown below.

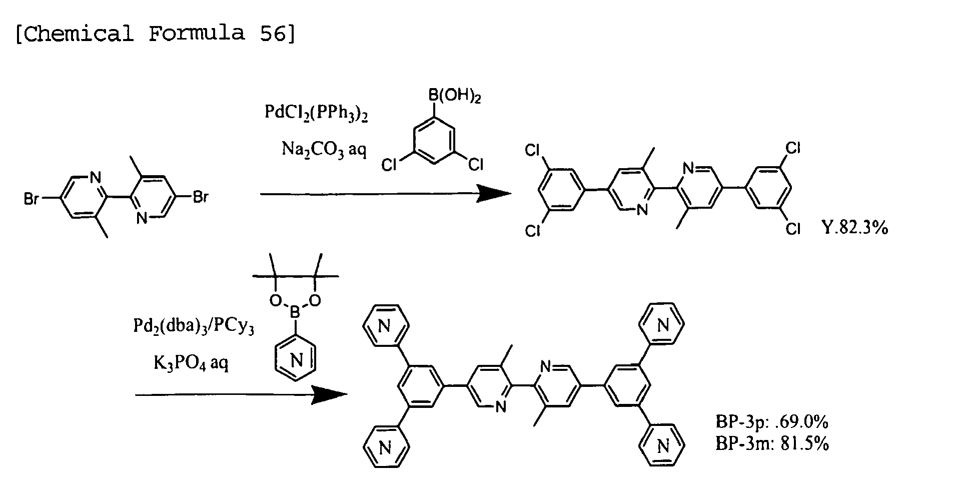

### (First Process: Synthesis of 5,5'-bis-(3,5-dichlorophenyl)-3,3'-dimethyl-[2,2'] bipyridinyl)

10.2 g (29.92 mmol) of 5,5'-dibromo-3,3'-dimethyl-[2,2'] pyridinyl synthesized by a known method (see Journal of Industrial and Engineering Chemistry, 8, p.103 (2002)), 24.8 g (104.7 mmol) of 3,5-dichlorophenylboric acid, 408 ml of toluene, 204 ml of ethanol, 66 ml of 2M sodium carbonate aqueous solution, and 2.1 g (2.995 mmol) of Pd(PPh₃)₂Cl₂ were placed and reacted in a nitrogen atmosphere for 11.5 hours.

The precipitated crystal was filtered, and washed with water and methanol. The filtrate was separated and a toluene layer was fractioned. The toluene layer was washed with water twice. After washing, a crude material obtained by collecting toluene was mixed with the filtered precipitate crystal. This crude material was subjected to a silica gel process, and the resulting crude material was recrystallized from toluene and purified.

Structure identification was performed by way of MS and ¹H-NMR, and it was confirmed that it was a target. Its yield was 11.6 g (82.3% yields).

### (Second Process: Synthesis of BP-3p)

3.5 g (7.381 mmol) of 5,5'-bis-(3,5-dichlorophenyl)-3,3'-dimethyl-[2,2'] bipyridinyl obtained by the above-mentioned first process, 10.6 g (51.66 mmol) of 4-(4,4,5,5-tetramethyl-[1, 3, 2]dioxaborolane-2-yl)- pyridine, 266 ml of 1,4-dioxane, 52 ml of 1.35M potassium phosphate aqueous solution, 0.68 g (0.7381 mmol) of Pd₂dba₃, and 0.52 g (1.845 mmol) of PCy₃ were placed in a reactor vessel in order and reacted in a nitrogen atmosphere for 24.0 hours at 85 °C.

The precipitated crystal was filtered, and its crude material was purified with a silica gel column using a developing solvent of chloroform-methanol mixture solvent.

Structure identification was performed by way of MS and ¹H-NMR, and it was confirmed that it was a target. Its yield was 3.3 g (69.0% yields).

Further, this was sublimed and purified, resulting in a melting point (DSC) of 435.70 °C.

### (Second Process: Synthesis of BP-3m)

4.0 g (8.435 mmol) of 5,5'-bis-(3,5-dichlorophenyl)-3,3'-dimethyl-[2,2'] bipyridinyl obtained by the above-mentioned first process, 12.1 g (59.05 mmol) of 3-(4,4,5,5-tetramethyl-[1, 3, 2]dioxaborolane-2-yl)- pyridine, 304 ml of 1,4-dioxane, 60 ml of 1.35M potassium phosphate aqueous solution, 0.77 g (0.8435 mmol) of Pd₂dba₃, and 0.59 g (2.109 mmol) of PCy₃ were placed in a reactor vessel in order and reacted in a nitrogen atmosphere for 24.0 hours at 85 °C.

The precipitated crystal was filtered, and its crude material was purified with a silica gel column using a developing solvent of chloroform-methanol mixture solvent.

Structure identification was performed by way of MS and ¹H-NMR, and it was confirmed that it was a target. Its yield was 4.4 g (81.5% yields).

Further, this was sublimed and purified, and its melting point (DSC) was 308.54 °C.

Further, in Examples 3 and 4, bipyridine derivatives expressed by the general formula as shown in the following (Chemical Formula 57) were synthesized.

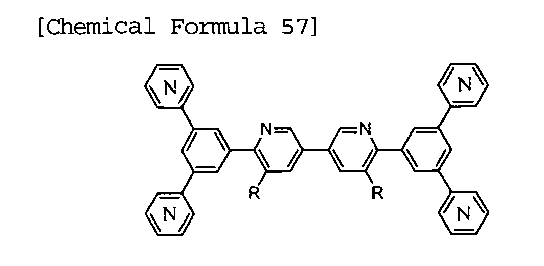

Of the bipyridine derivatives as shown in (Chemical Formula 57), one in which R=H and four terminal pyridyl groups are 4-pyridyl group is abbreviated and referred to as "BP-2p", one in which R=H and four terminal pyridyl groups are 3-pyridyl groups is abbreviated and referred to as "BP-2m", one in which R=Me(methyl group) and four terminal pyridyl groups are 4-pyridyl groups is abbreviated and referred to as "BP-4p", one in which R=Me(methyl group) and four terminal pyridyl groups are 3-pyridyl groups is abbreviated and referred to as "BP-4m", hereafter.

### [Example 3] (Synthesis of BP-2 series)

BP-2p and BP-2m were each synthesized by a synthesis scheme as shown below.

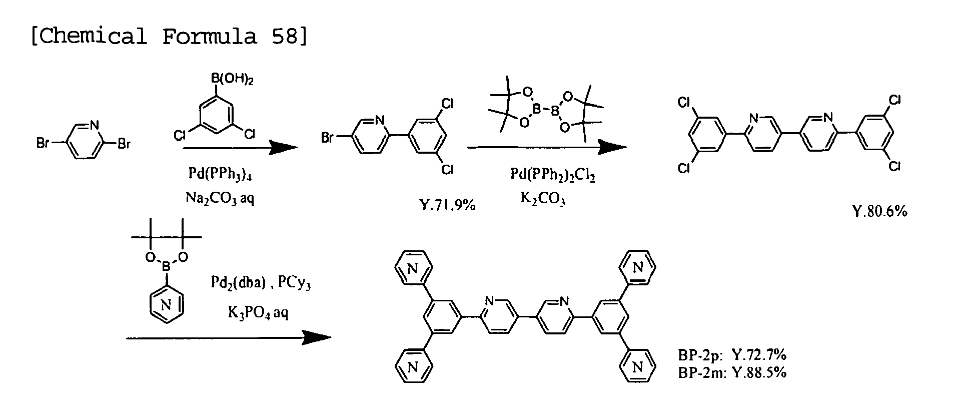

### (First Process: Synthesis of 5-bromo-2-(3,5-dichlorophenyl)-pyridine)

30.0 g (126.6 mmol) of 2,5-dibromopyridine, 24.6 g (129.1 mmol) of 3,5-dichlorophenylboric acid, 300 ml of toluene, 150 ml of ethanol, 130 ml of 2M sodium carbonate aqueous solution, and 7.3 g (6.33 mmol) of Pd(PPh₃)₄ were prepared and reacted in a nitrogen atmosphere for 17 hours.

Chloroform and water were poured into a reactor vessel and a chloroform layer was fractioned. The chloroform layer was washed with water twice. Its crude material obtained by collecting chloroform was purified with a silica gel column using a developing solvent of chloroform / n-hexane mixture solvent.

Structure identification was performed by way of MS and ¹H-NMR, and it was confirmed that it was a target. Its yield was 27.6 g (71.9% yields).

### (Second Process: Synthesis of 6,6'-bis-(3,5-dichlorophenyl)-[3,3'] bipyridinyl)

21.0 g (69.31 mmol) of 5-bromo-2-(3,5-dichlorophenyl)-pyridine synthesized by the above-mentioned first process, 9.2 g (36.04 mmol) of pinacoldiborane, 38.3 g (277.2 mmol) of potassium carbonate, 4.8 g (6.931 mmol) of Pd(PPh₃)₂Cl₂, and 500 ml of dimethylsulfoxide (DMSO) were prepared and reacted in a nitrogen atmosphere for 6 hours.

Chloroform was poured into a reactor vessel, and then a mineral salt was filtered. A crude material obtained by collecting DMSO was washed with water. The crude material was dried and subjected to a silica gel process, then recrystallized from toluene and purified.

Structure identification was performed by way of MS and ¹H-NMR, and it was confirmed that it was a target. Its yield was 12.5 g (80.6% yields).

### (Third Process: Synthesis of BP-2p)

3.0 g (6.724 mmol) of 6,6'-bis-(3,5-dichlorophenyl)-[3,3'] bipyridinyl synthesized by the above-mentioned second process, 10.0 g (47.07 mmol) of 4-(4,4,5,5-tetramethyl-[1, 3, 2]dioxaborolane-2-yl)-pyridine, 144 ml of 1,4-dioxane, 47 ml of 1.35M potassium phosphate aqueous solution, 0.62 g (0.6724 mmol) of Pd₂dba₃ , and 0.47 g (1.681 mmol) of PCy₃ were prepared and reacted in a nitrogen atmosphere for 41 hours.

The precipitated crystal was filtered, and washed with water and methanol. After drying, its crude material was purified with a silica gel column using a developing solvent of chloroform-methanol mixture solvent.

Structure identification was performed by way of MS and ¹H-NMR, and it was confirmed that it was a target. Its yield was 2.7 g (72.7% yields).

Further, this was sublimed and purified, and its melting point (DSC) was 396.23 °C.

### (Third Process: Synthesis of BP-2m)

4.0 g (8.965 mmol) of 6,6'-bis-(3,5-dichlorophenyl)-[3,3'] bipyridinyl synthesized by the above-mentioned second process, 12.8 g (62.76 mmol) of 3-(4,4,5,5-tetramethyl-[1, 3, 2]dioxaborolane-2-yl)- pyridine, 192 ml of 1,4-dioxane, 63 ml of 1.35M potassium phosphate aqueous solution, 0.82 g (0.8965 mmol) of Pd₂dba₃, and 0.63 g (2.241 mmol) of PCy₃ were prepared and reacted in a nitrogen atmosphere for 31.5 hours.

The precipitated crystal was filtered, and washed with water and methanol. After drying, its crude material was purified with a silica gel column using a developing solvent of chloroform-methanol mixture solvent.

Structure identification was performed by way of MS and ¹H-NMR, and it was confirmed that it was a target. Its yield was 4.8 g (88.5% yields).

Further, this was sublimed and purified, and its melting point (DSC) was 369.57 °C.

### [Example 4] (Synthesis of BP-4 series)

BP-4p and BP-4m were respectively synthesized by a synthesis scheme as shown below.

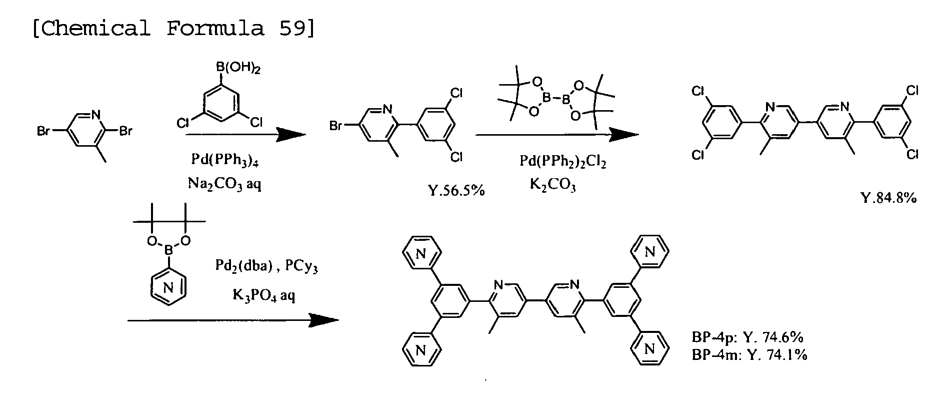

### (First Process: Synthesis of 5-bromo-2-(3,5-dichlorophenyl)-3-methylpyridine)

25.0 g (99.6 mmol) of 2,5-dibromo-3-picoline, 24.0 g (101.6 mmol) of 3,5-dichlorophenylboric acid, 288 ml of toluene, 144 ml of ethanol, 102 ml of 2M sodium carbonate aqueous solution, and 4.0 g (3.46 mmol) of Pd(PPh₃)₄ were prepared and reacted in a nitrogen atmosphere for 28.5 hours at 73 °C.

Chloroform and water were poured into a reactor vessel, and a chloroform layer was fractioned. The chloroform layer was washed with water twice. A crude material obtained by collecting chloroform was purified with a silica gel column using a developing solvent of chloroform / n-hexane mixture solvent.

Structure identification was performed by way of MS and ¹H-NMR, and it was confirmed that it was a target. Its yield was 17.9 g (56.5% yields).

### (Second Process: Synthesis of 6,6'-bis-(3,5-dichlorophenyl)-5,5'-dimethyl-[3,3'] bipyridinyl)

17.7 g (55.83 mmol) of 5-bromo-2-(3,5-dichlorophenyl)-3-methylpyridine synthesized by the above-mentioned first process, 7.4 g (29.03 mmol) of pinacoldiborane, 30.9 g (223.3 mmol) of potassium carbonate, 2.0 g (2.79 mmol) of Pd(PPh₃)₂Cl₂, and 414 ml of DMSO were prepared and reacted in a nitrogen atmosphere for 3 hours at 80 °C.

A mineral salt was filtered. A crude material obtained by collecting DMSO was washed with water. After drying, the crude material was recrystallized from toluene and purified.

Structure identification was performed by way of MS and ¹H-NMR, and it was confirmed that it was a target. Its yield was 11.2 g (84.8% yields).

### (Third Process: synthesis of BP-4p)

3.5 g (7.381 mmol) of 6,6'-bis-(3,5-dichlorophenyl)-5,5'-dimethyl-[3,3'] bipyridinyl synthesized by the above-mentioned second process, 10.6 g (51.66 mmol) of 4-(4,4,5,5-tetramethyl-[1, 3, 2]dioxaborolane-2-yl)- pyridine, 168 ml of 1,4-dioxane, 52 ml of 1.35M potassium phosphate aqueous solution, 0.68 g (0.7381 mmol) of Pd₂dba₃, and 0.52 g (1.845 mmol) of PCy₃ were prepared and reacted in a nitrogen atmosphere for 32.5 hours at 80 °C.

The precipitated crystal was filtered, and washed with water and methanol. After drying, its crude material was purified with a silica gel column using a developing solvent of chloroform-methanol mixture solvent.

Structure identification was performed by way of MS and ¹H-NMR, and it was confirmed that it was a target. Its yield was 3.55 g (74.6% yields).

Further, this was sublimed and purified, and its melting point (DSC) was 355.97 °C.

### (Third Process: Synthesis of BP-4m)

2.0 g (4.218 mmol) of 6,6'-bis-(3,5-dichlorophenyl)-5,5'-dimethyl-[3,3'] bipyridinyl synthesized by the above-mentioned second process, 6.0 g (29.52 mmol) of 3-(4,4,5,5-tetramethyl-[1, 3, 2]dioxaborolane-2-yl)- pyridine, 192 ml of 1,4-dioxane, 30 ml of 1.35M potassium phosphate aqueous solution, 0.39 g (0.4218 mmol) of Pd₂dba₃ , and 0.30 g (1.055 mmol) of PCy₃ were prepared and reacted in a nitrogen atmosphere for 48.0 hours at 80 °C.

The precipitated crystal was filtered, and washed with water and methanol. After drying, its crude material was purified with a silica gel column using a developing solvent of chloroform-methanol mixture solvent.

Structure identification was performed by way of MS and ¹H-NMR, and it was confirmed that it was a target. Its yield was 2.0 g (74.1% yields).

Further, this was sublimed and purified, and its melting point (DSC) was 368.58 °C.

Furthermore, in Example 5, a bipyridine derivative expressed by the general formula as shown in the following (Chemical Formula 60) was synthesized.

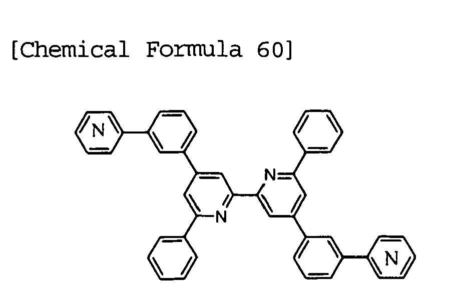

Of the bipyridine derivatives as shown in (Chemical Formula 60), one in which two terminal pyridyl groups are 4-pyridyl groups is abbreviated and referred to as "BP-5p", and one in which two terminal pyridyl groups are 3-pyridyl groups is abbreviated and referred to as "BP-5m", hereafter.

### [Example 5] (Synthesis of BP-5 series)

BP-5p and BP-5m were respectively synthesized by a synthesis scheme as shown below.

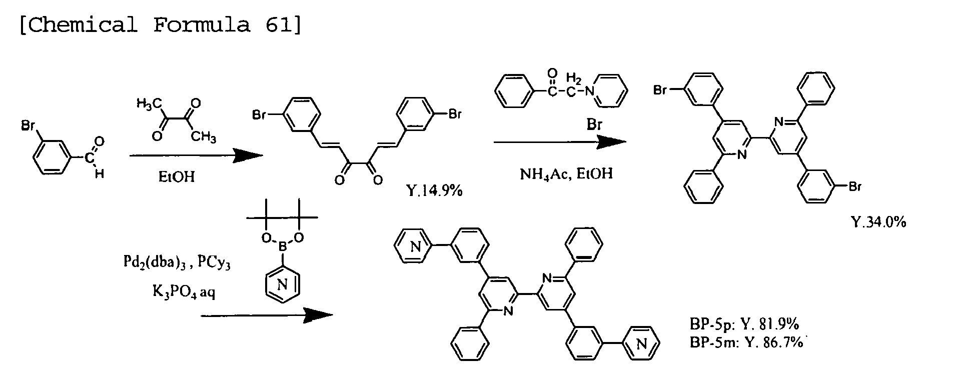

### (First Process: Synthesis of 1,6-bis-(3-bromophenyl)-hexa-1,5-diene-3,4-dione)

85 g (459.4 mmol) of 3-bromobenzaldehyde, 19.6 g (227.4 mmol) of diacetyl, 400 ml of ethanol, 1.9 g (22.74 mmol) of piperidine were placed in order and reacted in a nitrogen atmosphere for 21 hours.

A solution temperature was reduced to room temperature. The precipitated crystal was filtered and washed with methanol and n-hexane in order, to yield a target.

Structure identification was performed by way of MS. Its yield was 14.2 g (14.9% yields).

### (Second Process: Synthesis of 4,4'-bis-(3-bromophenyl)-6,6'-diphenyl-[2,2'] bipyridinyl)

14.2 g (33.80 mmol) of 1,6-bis-(3-bromophenyl)-hexa-1,5-diene-3,4-dione) synthesized by the above-mentioned first process, 18.8 g (67.60 mmol) of phenacylpyridinium bromide, 710 ml of ethanol, and 65.1 g (845.0 mmol) of ammonium acetate were placed in order and reacted in a nitrogen atmosphere for 8 hours.

A solution temperature was reduced to room temperature. The precipitated crystal was filtered and washed with water and methanol in order. The thus obtained crude material was recrystallized from toluene and purified.

Structure identification was performed in ¹H-NMR and MS, and it was confirmed that it was a target. Its yield was 7.1 g (34.0% yields).

### (Third Process: Synthesis of BP-5p)

3.0 g (4.852 mmol) of 4,4'-bis-(3-bromophenyl)-6,6'-diphenyl-[2,2'] bipyridinyl synthesized by the above-mentioned second process, 3.5 g (16.98 mmol) of 4-(4,4,5,5-tetramethyl-[1, 3, 2]dioxaborolane-2-yl)- pyridine, 230 ml of 1,4-dioxane, 17 ml of 1.35M potassium phosphate aqueous solution, 0.44 g (0.485 mmol) of Pd₂(dba)₃, and 0.34 g (1.213 mmol) of PCy₃ were placed in order and reacted in a nitrogen atmosphere for 6 hours.

A solution temperature was reduced to room temperature. The precipitated crystal was filtered and washed with methanol and n-hexane in order. The thus obtained crude material was purified with a silica gel column using a developing solvent of chloroform-methanol mixture solvent.

Structure identification was performed by way of MS and ¹H-NMR, and it was confirmed that it was a target. Its yield was 2.4 g (81.9% yields).

Further, this was sublimed and purified, and its melting point (DSC) was 280.02 °C.

### (Third Process: Synthesis of BP-5m)

Instead of 4-(4,4,5,5-tetramethyl-[1, 3, 2]dioxaborolane-2-yl)- pyridine, 3-(4,4,5,5-tetramethyl-[1, 3, 2]dioxaborolane-2-yl)-pyridine was used. Other conditions were the same as those for BP-5p and a reaction, post-processing, and purification were carried out.

Structure identification was performed by way of MS and ¹H-NMR, and it was confirmed that it was a target. Its yield was 2.6 g (86.7% yields).

Further, this was sublimed and purified, and its melting point (DSC) was 289.44 °C.

In Example 6, a bipyridine derivative expressed by the general formula as shown in the following (Chemical Formula 62) was synthesized.

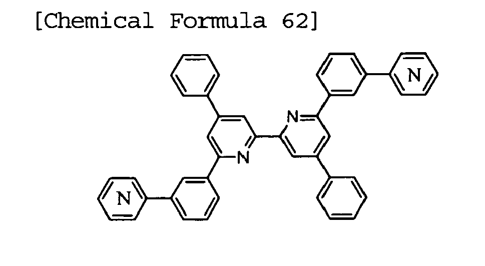

Of the bipyridine derivatives as shown in (Chemical Formula 62), one in which two terminal pyridyl groups are 4-pyridyl groups is abbreviated and referred to as "BP-6p", and one in which two terminal pyridyl groups are 3-pyridyl groups is abbreviated and referred to as "BP-6m", hereafter.

### [Example 6] (Synthesis of BP-6 series)

BP-6p and BP-6m were respectively synthesized by a synthesis scheme as shown below.

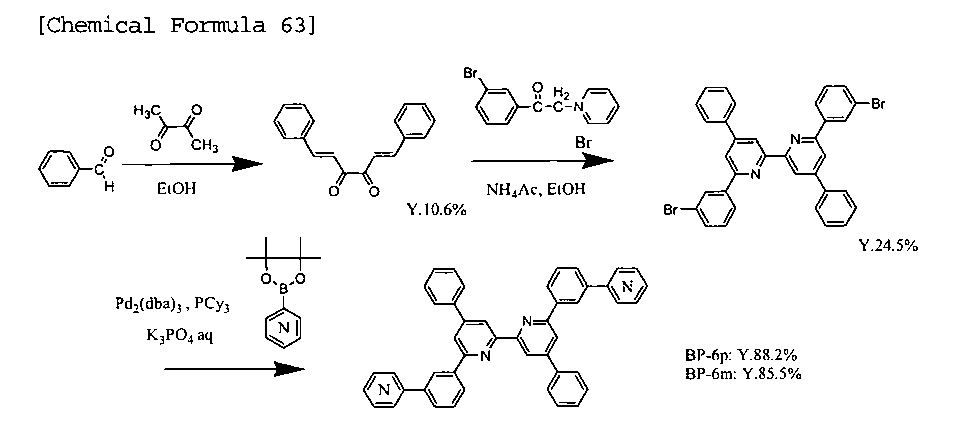

### (First Process: Synthesis of 1,6-diphenyl-hexa-1,5-diene-3,4-dione)

23.2 g (218.2 mmol) of bromobenzaldehyde, 9.3 g (108.0 mmol) of diacetyl, 186 ml of ethanol, and 0.9 g (10.80 mmol) of piperidine were placed in order and reacted in a nitrogen atmosphere for 5 hours.

A solution temperature was reduced to room temperature. The precipitated crystal was filtered and washed with methanol and n-hexane in order, to yield a target.

Structure identification was performed by way of MS. Its yield was 3.0 g (10.6% yields).

### (Second Process: Synthesis of 6,6'-bis-(3-bromophenyl)-4,4'-diphenyl-[2,2'] bipyridinyl)

8.0 g (30.50 mmol) of 1,6-diphenyl-hexa-1,5-diene-3,4-dione synthesized by the above-mentioned first process, 21.8 g (61.00 mmol) of 3'-bromo phenacylpyridinium bromide, 545 ml of ethanol and 58.8 g (762.5 mmol) of ammonium acetate were placed in order and reacted in a nitrogen atmosphere for 9 hours.

A solution temperature was reduced to room temperature. The precipitated crystal was filtered and washed with water and methanol in order. The thus obtained crude material was recrystallized from toluene and purified.

Structure identification was performed by ¹H-NMR and MS, and it was confirmed that it was a target. Its yield was 4.6 g (24.5% yields).

### (Third Process: Synthesis of BP-6p)

2.2 g (3.558 mmol) of 6,6'-bis-(3-bromophenyl)-4,4'-diphenyl-[2,2'] bipyridinyl synthesized by the above-mentioned second process, 2.6 g (12.45 mmol) of 4-(4,4,5,5-tetramethyl-[1, 3, 2]dioxaborolane-2-yl)- pyridine, 168 ml of 1,4-dioxane, 12.5 ml of 1.35M potassium phosphate aqueous solution, 0.33 g (0.355 mmol) of Pd₂(dba)₃ and 0.25 g (0.889 mmol) of PCy₃ were placed in order and reacted in a nitrogen atmosphere for 6 hours.

A solution temperature was reduced to room temperature. The precipitated crystal was filtered and washed with methanol and n-hexane in order. The thus obtained crude material was purified with a silica gel column using a developing solvent of chloroform-methanol mixture solvent.

Structure identification was performed by way of MS and ¹H-NMR, and it was confirmed that it was a target. Its yield was 1.94 g (88.2% yields).

Further, this was sublimed and purified, and its melting point (DSC) was 322.92 °C.

### (Third Process: Synthesis of BP-6m)

Instead of 4-(4,4,5,5-tetramethyl-[1, 3, 2]dioxaborolane-2-yl)- pyridine, 3-(4,4,5,5-tetramethyl-[1, 3, 2]dioxaborolane-2-yl)-pyridine was used. Other conditions were the same as those for BP-6p and a reaction, post-processing, and purification were carried out.

Structure identification was performed by way of MS and ¹H-NMR, and it was confirmed that it was a target. Its yield was 1.88 g (85.5% yields).

Further, this was sublimed and purified, and its melting point (DSC) was 291.05 °C.

Further, a bipyridine derivative expressed by the following general formula (Chemical Formula 64) was synthesized in Example 7.

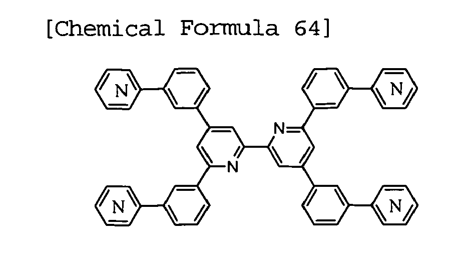

Of the bipyridine derivatives as shown in (Chemical Formula 64), one in which four terminal pyridyl groups are 4-pyridyl groups is abbreviated and referred to as "BP-7p", and one in which four terminal pyridyl groups are 3-pyridyl groups is abbreviated and referred to as "BP-7m", hereafter.

### [Example 7] (Synthesis of BP-7 series)

BP-7p and BP-7m were synthesized respectively by synthesis schemes as shown beow.

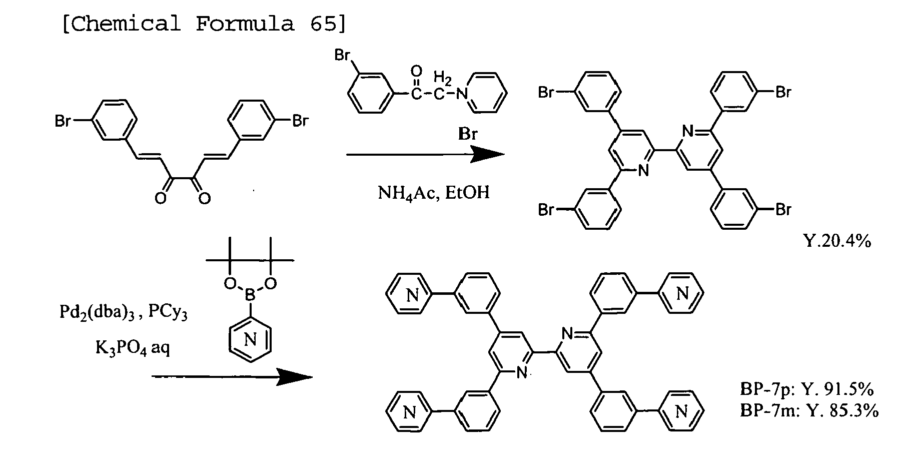

### (Fist Process: Synthesis of 4,6,4',6'-tetrakis-(3-bromophenyl)-[2,2']bipyridinyl)

8.2 g (19.61 mmol) of 1,6-bis-3-(bromophenyl)-hexa-1,5-diene-3,4-dione and 14.0 g (39.21 mmol) of 3'-bromophenacylpyridinium bromide were used. Other conditions were the same as those for the second process of BP-5 series, and a reaction, post-processing, and purification were carried out.

Structure identification was performed by way of ¹H-NMR and MS, and it was confirmed that it was a target. Its yield was 3.1 g (20.4% yields).

### (Second Process: Synthesis of BP-7p)

3.0 g (3.865 mmol) of 4,6,4',6'-tetrakis-(3-bromophenyl)-[2,2']bipyridinyl as synthesized in the above-mentioned first process and 5.5 g (27.06 mmol) of 4-(4,4,5,5-tetramethyl-[1,3,2]dioxaborolane-2-yl)-pyridine were used. Other conditions were the same as those for BP-5p, and a reaction, post-processing, and purification were carried out.

Structure identification was performed by way of ¹H-NMR and MS, and it was confirmed that it was a target. Its yield was 2.7 g (91.5% yields).

Further, this was sublimed, and its melting point (DSC) was 346.62°C.

### (Second Process: Synthesis of BP-7m)

Instead of 4-(4,4,5,5-tetramethyl-[1,3,2]dioxaborolane-2-yl)-pyridine, 3-(4,4,5,5-tetramethyl-[1,3,2] dioxaborolane-2-yl)-pyridine was used.
Other conditions were the same as those for BP-5p, and a reaction, post-processing, and purification were carried out.

Structure identification was performed by way of ¹H-NMR and MS, and it was confirmed that it was a target. Its yield was of 2.2 g (85.3% yields).

Further, this was sublimed, its melting point (DSC) was 220.55°C, and Tg (DSC) was 93.78°C.

Further, a bipyridine derivative expressed by the following general formula (Chemical Formula 66) was synthesized in Example 8.

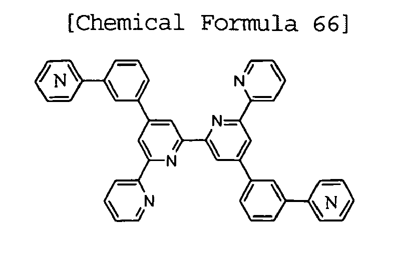

Of the bipyridine derivatives as shown in the following (Chemical Formula 66), one in which two terminal pyridyl groups are 4-pyridyl groups is abbreviated and referred to as "BP-8p", and one in which two terminal pyridyl groups are 3-pyridyl groups is abbreviated and referred to as "BP-8m", hereafter.

### [Example 8] (Synthesis of BP-8 series)

BP-8p and BP-8m were respectively synthesized by a synthesis scheme as shown below.

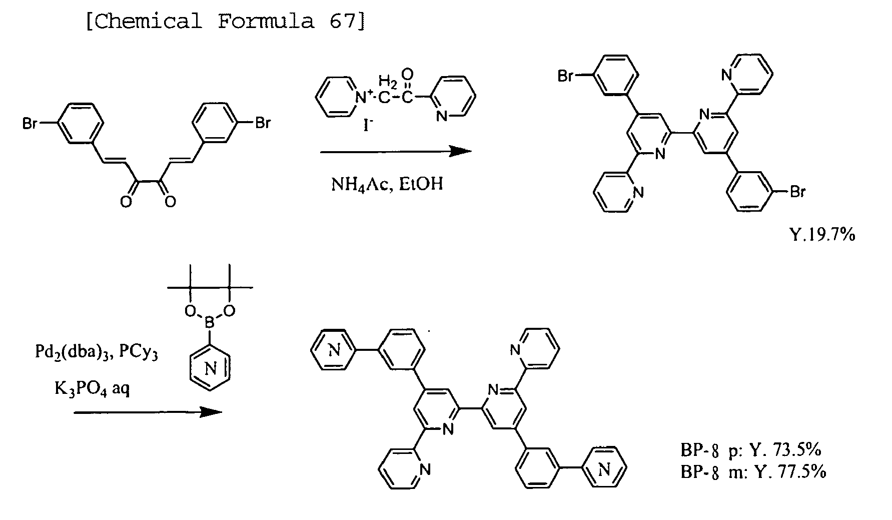

### (First Process: Synthesis of 4,4'-bis-(3-bromophenyl)-6,6'-bis-(pyridine-2-yl)-[2,2'] bipyridinyl)

16.0 g (38.09 mmol) of 1,6-bis-3-(bromophenyl)-hexa-1,5-diene-3,4-dione and 26.1 g (79.99 mmol) of 1-[2-oxo-(2-pyridine-2-yl)ethylpyridinium iodide were used. Other conditions were the same as those for the second process of BP-5 series, and a reaction and post-processing were carried out. After the post-processing, dispersion cleaning was performed using DMF.

Structure identification was performed by way of MS, and it was confirmed that it was a target. Its yield was 4.6 g (19.7% yields).

### (Second Process: Synthesis of BP-8p)

3.0 g (4.836 mmol) of 4,4'-bis-(3-bromophenyl)-6,6'-bis-(pyridine-2-yl)-[2,2'] bipyridinyl synthesized in the above-mentioned first process and 3.5 g (16.93 mmol) of 4-(4,4,5,5-tetramethyl-[1,3,2]dioxaborolane-2-yl)-pyridine were used. Other conditions were the same as those for BP-5p, and a reaction, post-processing, and purification were carried out.

Structure identification was performed by way of ¹H-NMR and MS, and it was confirmed that it was a target. Its yield was of 2.2 g (73.5% yields).

Further, this was sublimed, and its melting point (TG-DTA) was 298.6°C.

### (Second Process: Synthesis of BP-8m)

Instead of 4-(4,4,5,5-tetramethyl-[1,3,2]dioxaborolane-2-yl)-pyridine, 3-(4,4,5,5-tetramethyl-[1,3,2]dioxaborolane-2-yl)-pyridine was used. Other conditions were the same as those for BP-5p, and a reaction, post-processing, and purification were carried out.

Structure identification was performed by way of ¹H-NMR and MS, and it was confirmed that it was a target. Its yield was 1.0 g (77.5% yields).

Further, this was sublimed, and its melting point (TG-DTA) was 306.3°C.

### (Preparation of White Phosphorescence Element)

Of the bipyridine derivatives each synthesized in Examples 1-7 above, BP-1 series, BP-2 series, and BP-4 to BP-6 series were used for electron transport layers to make white phosphorescence elements.

In addition, BTPS as shown in the following (Chemical Formula 68) was used for hole inhibition layers, and NS21 (available from Nippon Steel Chemical Co., Ltd.) and KLHT03 (available from Chemipro Kasei Kaisha, Ltd.) were used as hole transport materials.

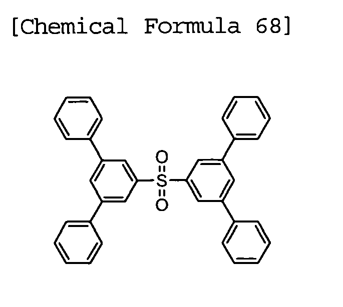

A layer structure of the thus prepared element may be simplified and shown as being "ITO (110nm) / NS21:MoO3 (13.6 nm, 20%) / KLHT03:Mo(5nm, 20%) / KLHT03 (15 nm) / TCTA:Firpic (4.5 nm, 20%) / Pq₂Ir (dpm) (1 nm, 5%) / Firpic (4.5 nm, 20%) / BTPS (8 nm) / electron transport layer (58 nm) / Liq (1 nm) / Al (100 nm)".

In addition, numerical values in the above parentheses show film thicknesses (nm) and dopant concentrations (%).

A particular method of making the elements is as follows.

First, a glass substrate having formed thereon a patterned transparent electroconductive film (ITO) with a film thickness of 110 nm was subjected to washing treatments in the order of ultrasonic cleaning by pure water and a surfactant, washing with flowing pure water, ultrasonic cleaning by a 1:1 mixed solution of pure water and isopropyl alcohol, and boiling washing by isopropyl alcohol. This substrate was slowly pulled up from the boiling isopropyl alcohol, and dried in isopropyl alcohol vapor, and, finally ultraviolet ozone cleaning was performed.

This substrate having an ITO electrode (anode) was placed in a vacuum chamber which was evacuated to 1×10⁻⁶ Torr. In this vacuum chamber, each molybdenum boat filled up with a vapor deposition material and a vapor deposition mask for forming a film in a predetermined pattern were placed, the above-mentioned boat was electrically heated, and the vapor deposition material was evaporated to thereby form a layer structure as described above one by one.

Finally, while maintaining the vacuum chamber at a vacuum, masks were replaced to install masks for cathode vapor deposition, so as to form a cathode of an aluminum (Al) layer.

The vacuum chamber was returned to ambient pressure, and the substrate on which each layer was deposited as described above was taken out, and it was moved in a glove box in which nitrogen substitution was carried out. By using a UV curing resin, it was sealed with another glass board to obtain an organic EL element.

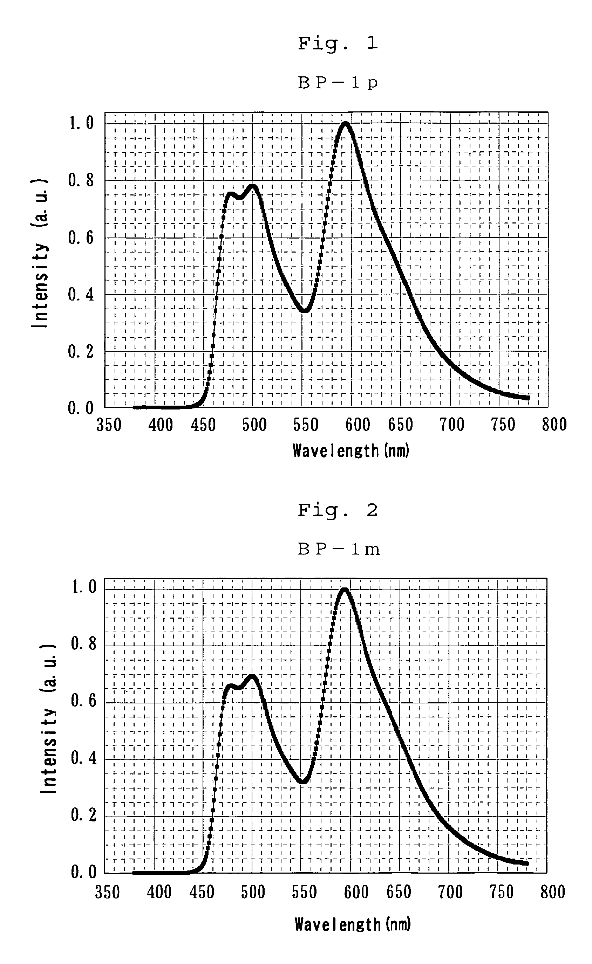

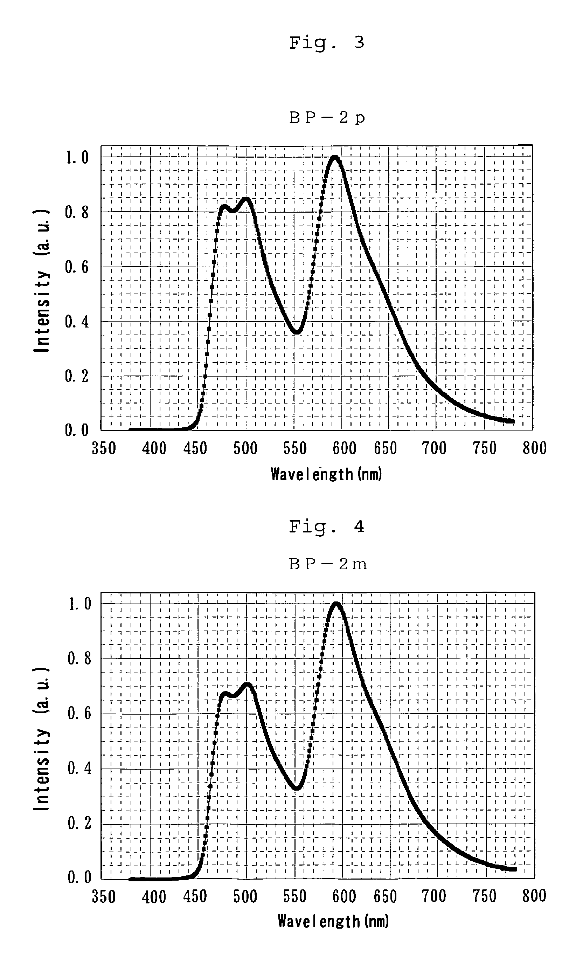

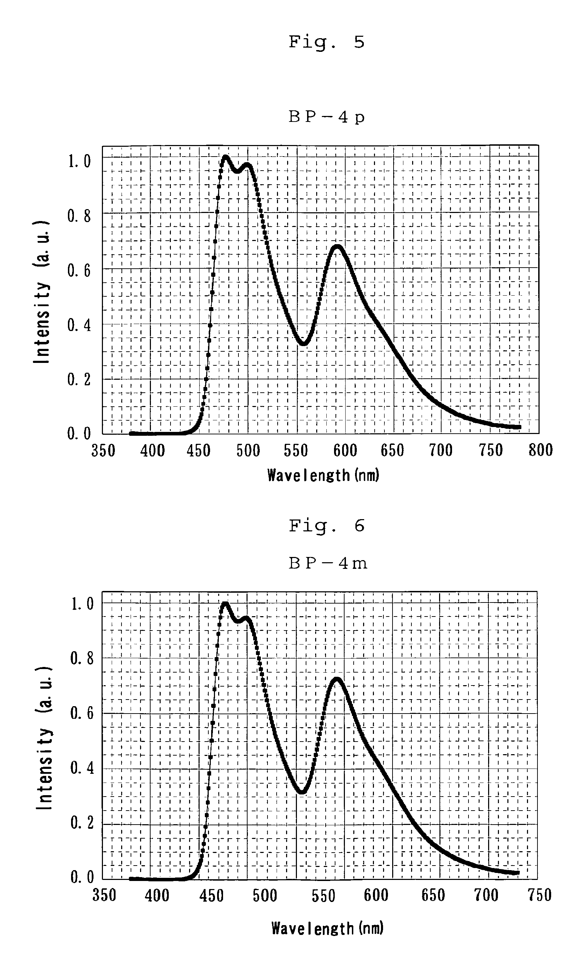

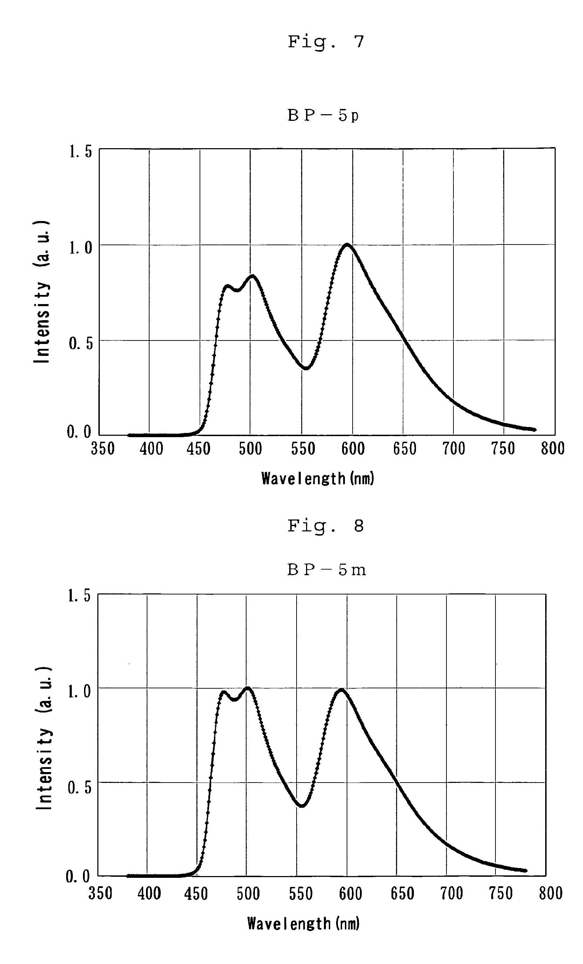

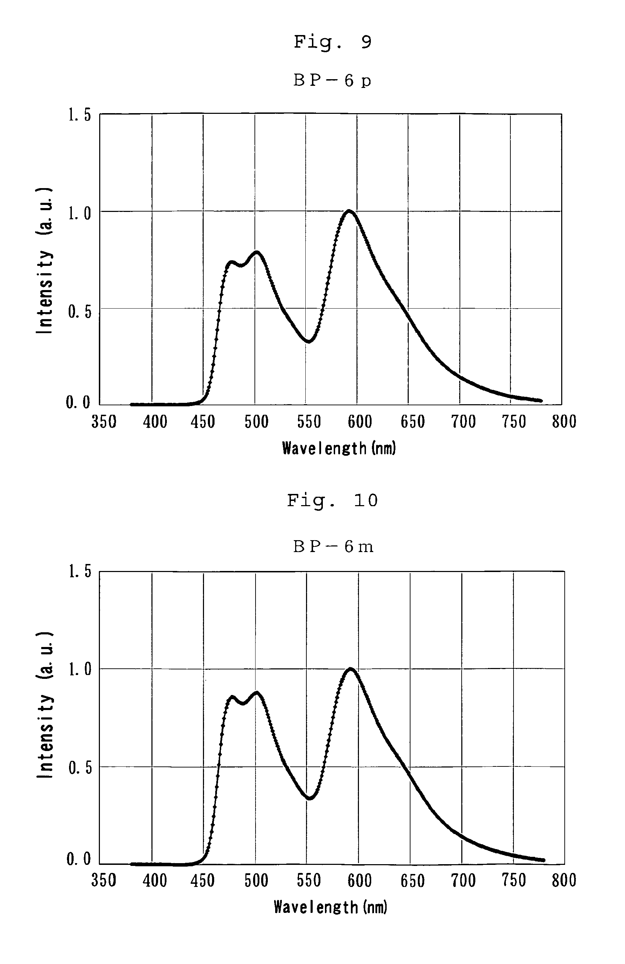

Efficiency of each white phosphorescence element as prepared above was evaluated. The efficiencies in the case of current densities 1, 2.5, and 25 A/m² are collectively shown in Table 1 and 2. Further, emission spectra at a current density of 2.5 A/m² are shown in FIGS. 1-10.

**[Table 1]**

| Electron Transport Layer | | BP-1p | BP-1m | BP-2p | BP-2m | BP-4p | BP-4m |
|---|---|---|---|---|---|---|---|
| External Quantum Efficiency (%) | 1A/m² | 11.7 | 18.2 | 13.6 | 18.2 | 14.8 | 19.4 |
| | 2.5A/ m² | 14.3 | 17.6 | 14.5 | 17.5 | 15.5 | 18.4 |
| | 25A/m² | 12.4 | 15.5 | 12.7 | 15.9 | 14.2 | 11.9 |
| Luminous Efficiency (1m/W) | 1A/m² | 21.3 | 39.1 | 26.9 | 39.8 | 28.8 | 39.2 |
| | 2.5A/ m² | 22.5 | 35.4 | 26.7 | 36.8 | 28.1 | 34.1 |
| | 25A/m² | 14.6 | 25.3 | 19.1 | 28.9 | 21.5 | 17.9 |
| Energy Conversion Efficiency (%) | 1A/m² | 7.1 | 13.1 | 9.1 | 13.3 | 9.9 | 13.5 |
| | 2.5A/ m² | 7.6 | 11.8 | 9.0 | 12.3 | 9.7 | 18.4 |
| | 25A/m² | 4.9 | 8.5 | 6.4 | 9.7 | 7.4 | 6.3 |

**[Table 2]**

| Electron Transport Layer | | Bp-5p | BP-5m | BP-6p | BP-6m |
|---|---|---|---|---|---|
| External Quantum Efficiency (%) | 1A/m² | 18.6 | 17.0 | 19.1 | 17.0 |
| | 2.5A/m² | 18.3 | 17.1 | 18.9 | 17.1 |
| | 25A/m² | 16.2 | 15.8 | 17.1 | 16.0 |
| Luminous Efficiency (lm/W) | 1A/m² | 36.0 | 34.5 | 43.1 | 36.7 |
| | 2.5A/m² | 32.3 | 31.5 | 40.3 | 33.9 |
| | 25A/m² | 21.5 | 21.7 | 29.5 | 23.5 |
| Energy Conversion Efficiency (%) | 1A/m² | 12.3 | 12.0 | 14.5 | 12.5 |
| | 2.5A/m² | 11.1 | 11.0 | 13.6 | 11.6 |
| | 25A/m² | 7.4 | 7.6 | 10.0 | 8.1 |

Any of the above-mentioned elements provided white luminescence.

From these results, it is recognized that any of BP-1, 2, 4-6 functions as the electron transport layer and the electron injection layer.

### (Preparation of Blue Phosphorescence Element)

Of the bipyridine derivatives each synthesized in Examples 1-7 above, BP-5 to 7 series were used for the electron transport layer to make a blue phosphorescence element.

A layer structure of the thus prepared element may be simplified and shown as being "ITO (110nm) / TAPC: MoO₃ (30 nm, 10%) / TCTA:Firpic (5 nm, 7%) / TCTA:Firpic (5 nm, 20%) / electron transport layer (50 nm) / LiF (0.5 nm) / Al (100 nm)".

In addition, numerical values in the above parentheses show film thicknesses (nm) and dopant concentrations (%).

### [Comparative Example 1]

Instead of BP-5 to 7 series, a compound (BMPyPB) as shown in the following (Chemical Formula 69) was used for the electron transport layer. Other conditions were the same as those for Examples above, and a blue phosphorescence element was prepared.

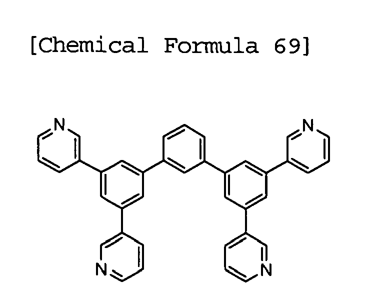

Efficiency of each blue phosphorescence element as prepared above was evaluated. The efficiencies in the case of current densities:1, 2.5, 10, and 25 A/m² are collectively shown in Table 3.

Further, direct current (25 A/m²) was applied to an element to be continuously lit at room temperature and attenuation time of emission brightness decreasing to 70% of initial brightness was measured. This result is also collectively shown in Table 3. In addition, the evaluation result of this attenuation time was expressed by a relative value, assuming that of a time unit of the element in Comparative Example 1 is 100.

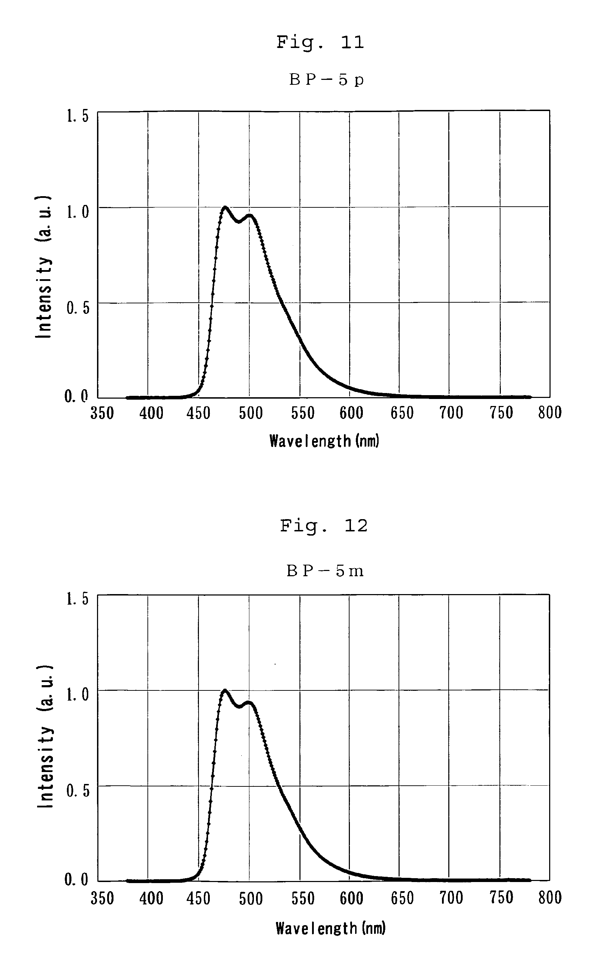

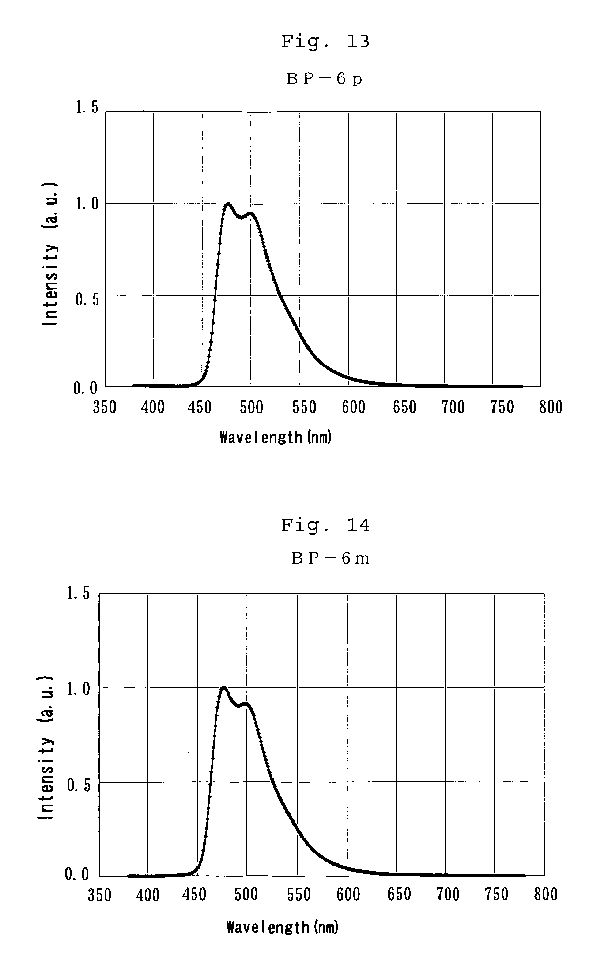

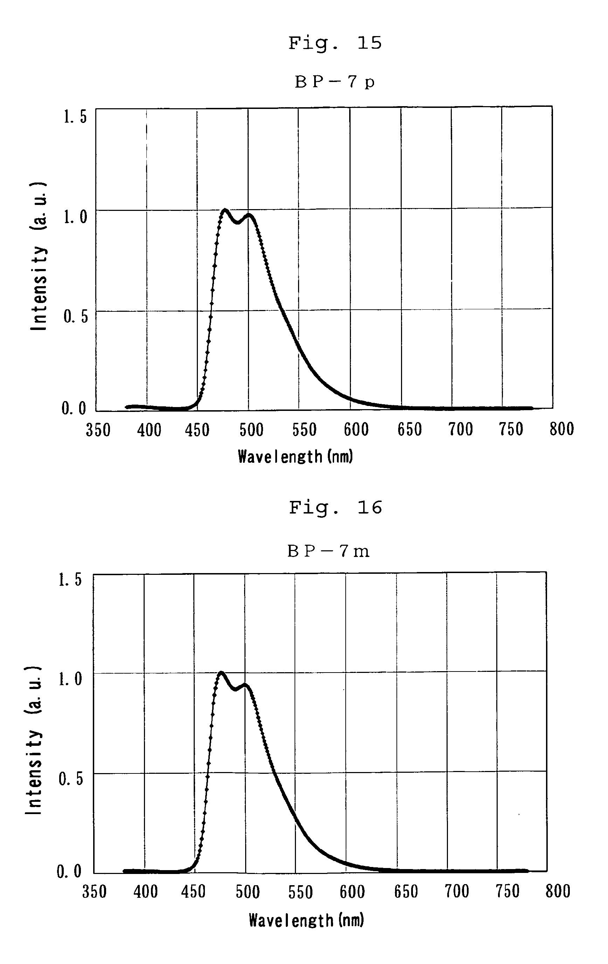

Furthermore, emission spectra of the elements in the case of 10 A/m² are shown in FIGS. 11 to 17.

**[Table 3]**

| External Quantum Efficiency (%) | | BP-5p | BP-5m | BP-6p | BP-6m | BP-7p | BP-7m | BMPyPB |
|---|---|---|---|---|---|---|---|---|
| External Quantum Efficiency (%) | 1A/m² | 15.3 | 17.2 | 12.0 | 15.4 | 6.8 | 11.8 | 20.3 |
| | 2.5A/m² | 15.1 | 16.8 | 11.9 | 14.9 | 7.4 | 11.8 | 18.7 |
| | 10A/m² | 14.3 | 15.9 | 11.5 | 13.8 | 7.8 | 11.4 | - |
| | 25A/m² | 13.0 | 14.5 | 10.6 | 12.4 | 7.4 | 10.6 | - |
| Luminous Efficiency (1M/W) | 1A/m² | 38.0 | 41.6 | 29.3 | 35.2 | 18.5 | 30.3 | 43.4 |
| | 2.5A/m² | 35.4 | 38.5 | 28.0 | 32.5 | 19.5 | 29.2 | 37.7 |
| | 10A/m² | 29.7 | 32.5 | 24.5 | 26.9 | 19.3 | 26.0 | - |
| | 25A/m² | 24.1 | 26.6 | 20.4 | 21.6 | 17.0 | 22.2 | - |
| Energy Conversion Efficiency (%) | 1A/m² | 13.7 | 15.3 | 10.8 | 13.3 | 6.6 | 11.0 | 15.8 |
| | 2.5A/m² | 12.8 | 14.2 | 10.3 | 12.3 | 7.0 | 10.7 | 13.8 |
| | 10A/m² | 10.7 | 12.0 | 9.0 | 10.2 | 6.9 | 9.6 | - |
| | 25A/m² | 8.7 | 9.8 | 7.5 | 8.2 | 6.1 | 8.2 | - |
| 70% Attenuation time | 25A/m² | 2000 | 3500 | 2300 | 1750 | 1100 | 750 | 100 |

Each of the elements using the above-mentioned BP-5 to 7 gave light blue luminescence originated from FIrpic in each emission spectrum. It is therefore confirmed that BP-5 to 7 can be applied to the blue phosphorescence element.

Further, as can be seen from Table 3, it is confirmed that each of the elements using BP-5 and 6 allows an efficiency with a level equivalent to or greater than that of the element (Comparative example 1) using MPyPB in the case of 1 A/m² and 2.5 A/m².

In addition, due to degradation of the element, it was not able to evaluate the element using BMPyPB in the case of 10 A/m² and 25 A/m². One reason for this is that because the element using BMPyPB may be unstable.

Further, as for the attenuation time of brightness decreasing to 70%, it is confirmed that the element using BP-5 to 7 is improved much better than the element using BMPyPB. Thus, it is thought that the element using BP-5 to 7 is more excellent in stability than the element using BMPyPB.

As described above, it can be said that BP-5 to 7 are more excellent in the performance as an electronic transport material than BMPyPB is.

### (Preparation of Single Carrier Element)

The bipyridine derivatives each synthesized in Examples 1-7 above, any of BP-1 to 7 series, or BMPyPB used in Comparative Example 1 above were used for electron transport layers to make electronic elements (singe carrier elements).

A layer structure of the thus prepared element may be simplified and shown as being ITO (110nm) / electron transport layer (100 nm) / Liq (1.0 nm) / Al (100 nm).

In addition, numerical values in the above parentheses show film thicknesses (nm) and dopant concentrations (%).

An ITO side of the electronics element (single carrier element) prepared in the above was used as an anode, and an Al side was used as a cathode, then direct current was supplied to evaluate a current density / voltage characteristic.

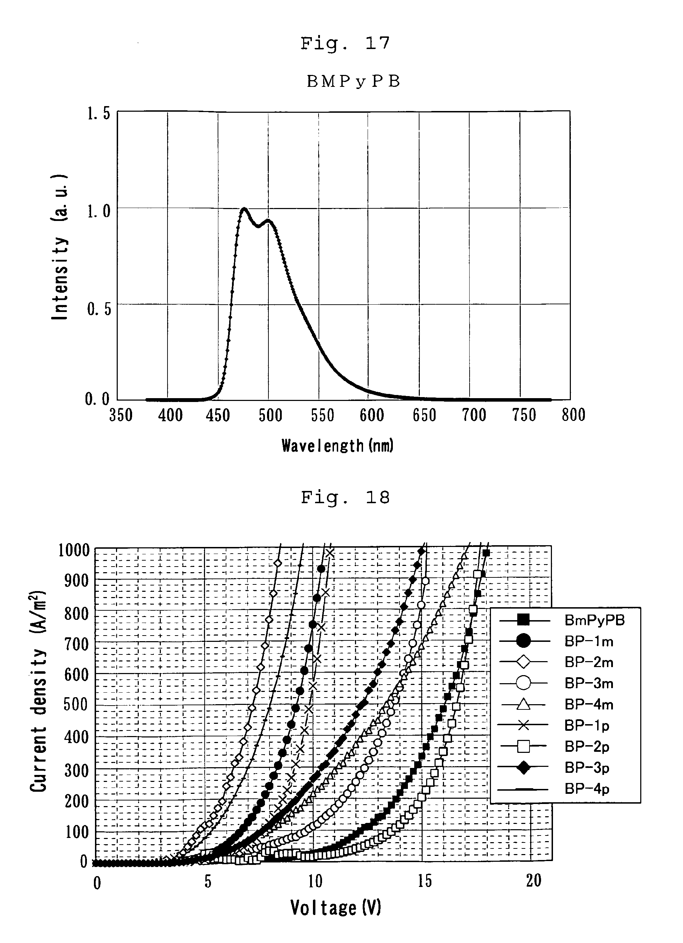

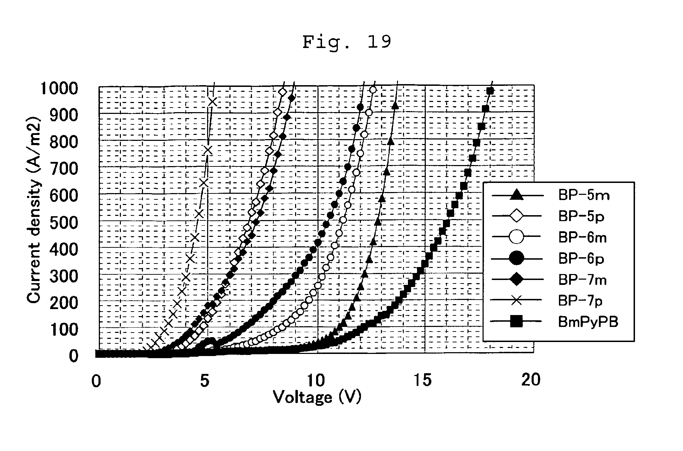

FIGS. 18 and 19 show current density / voltage characteristics. FIG. 18 shows BP-1 to 4 and BmPyPB, and FIG. 19 shows BP-5 to 7 and BmPyPB. In addition, during evaluation measurement, luminescence from each element was not observed, and the resulting characteristics reflect only electron current.

It can be seen from the evaluation results shown in FIGS. 18 and 19 that each of the bipyridine derivatives of BP-1 to 7 functions as an electron transport material. Further, as for the characteristic of the electronics element using each of the bipyridine derivatives of BP-1 to 7, the element using BP-2p is equivalent to the element using BmPyPB. It is confirmed that the element using each of other bipyridine derivatives is much more of low voltage.

As such, it can be said that the bipyridine derivatives of BP-1 to 7 are more excellent in the performance as an electronic transport material than BMPyPB is.

## Claims

1. A bipyridine derivative expressed by the following general formula (1). 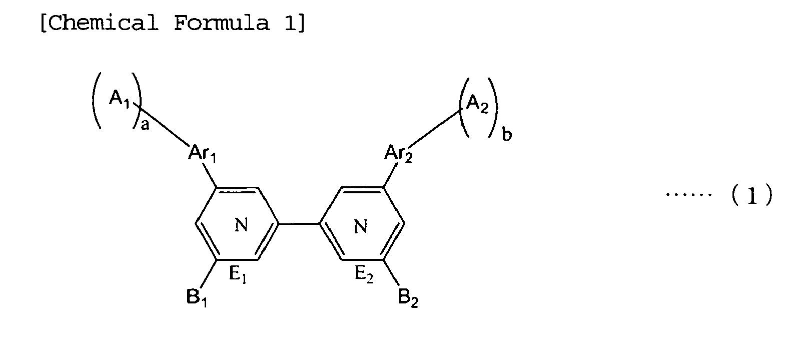 (where A₁ and A₂ each independently represent a substituted or unsubstituted aromatic heterocyclic group (except for a carbazolyl group) and may be a substituent in which an aromatic hydrocarbon group and the aromatic heterocyclic group are combined; a and b are each independently 1 or 2; Ar₁ and Ar₂ each independently represent a divalent or trivalent aromatic hydrocarbon group, and said hydrocarbon group may each be independently substituted by hydrogen atom, an alkyl group, a cycloalkyl group, or an alkoxy group; B₁ and B₂ each independently represent hydrogen atom, an alkyl group, a cycloalkyl group, an alkoxy group, a substituted or unsubstituted aromatic hydrocarbon group, or a substituted or unsubstituted aromatic heterocyclic group; and a ring E₁ and a ring E₂ represent a pyridine ring, and may each independently have a substituent selected from hydrogen atom, an alkyl group, a cycloalkyl group, and an alkoxy group.)

2. The bipyridine derivative as claimed in claim 1, being expressed by the following general formula (2). 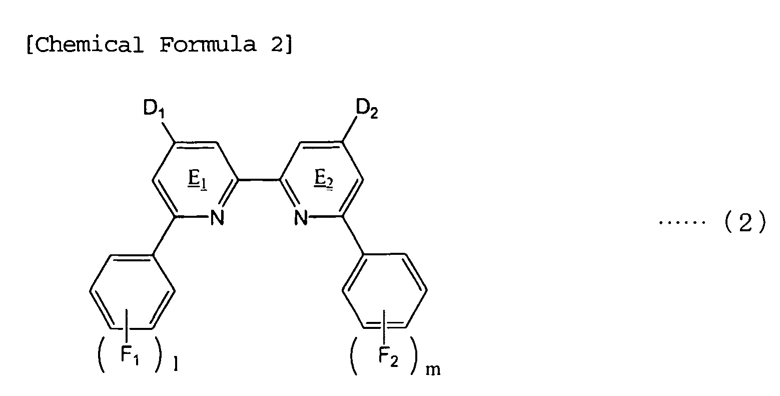 (where F₁ and F₂ each independently represent a substituted or unsubstituted aromatic heterocyclic group (except for a carbazolyl group) and may be a substituent in which an aromatic hydrocarbon group and an aromatic heterocyclic group are combined; any hydrogen atom on benzene ring may be substituted by an alkyl group, a cycloalkyl group, or an alkoxy group; 1 and m are each independently 1 or 2; D₁ and D₂ each independently represent hydrogen atom, an alkyl group, a cycloalkyl group, an alkoxy group, a substituted or unsubstituted aromatic hydrocarbon group, or a substituted or unsubstituted aromatic heterocyclic group; and a ring E₁ and a ring E₂ represent a pyridine ring and may each independently have a substituent selected from hydrogen atom, an alkyl group, a cycloalkyl group, and an alkoxy group.)

3. The bipyridine derivative as claimed in claim 1, being expressed by the following general formula (3). 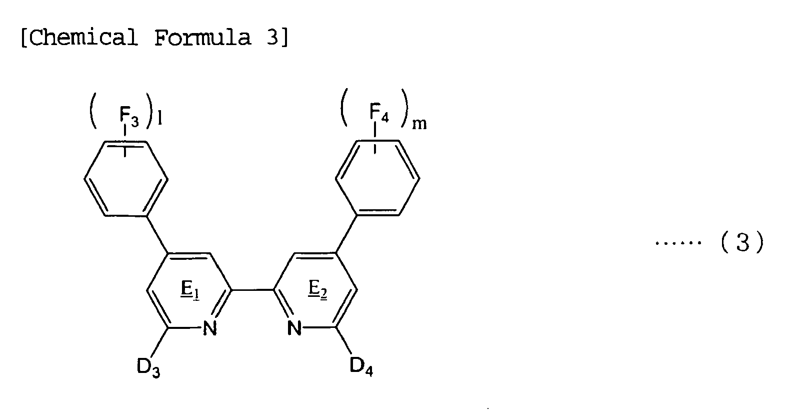 (where F₃ and F₄ each independently represent a substituted or unsubstituted aromatic heterocyclic group (except for a carbazolyl group) and may be a substituent in which an aromatic hydrocarbon group and an aromatic heterocyclic group are combined; any hydrogen atom on benzene ring may be substituted by an alkyl group, a cycloalkyl group, or an alkoxy group; 1 and m are each independently 1 or 2; D₃ and D₄ each independently represent hydrogen atom, an alkyl group, a cycloalkyl group, an alkoxy group, a substituted or unsubstituted aromatic hydrocarbon group, or a substituted or unsubstituted aromatic heterocyclic group; and a ring E₁ and a ring E₂ represent a pyridine ring and may each independently have a substituent selected from hydrogen atom, an alkyl group, a cycloalkyl group, and an alkoxy group.)

4. A bipyridine derivative expressed by the following general formula (4). 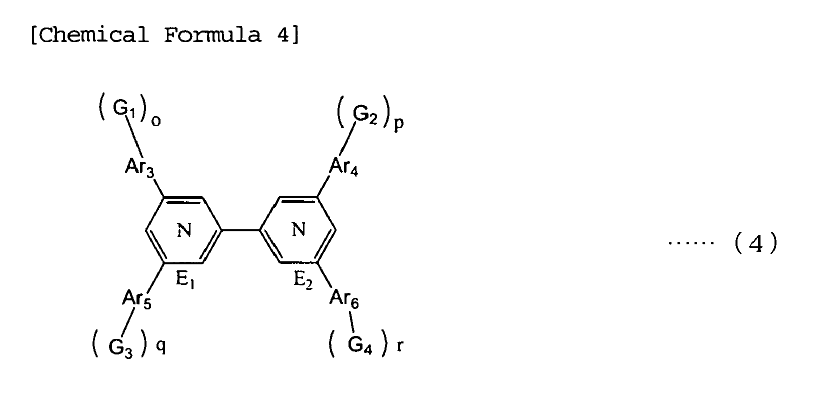 (where G₁- G₄ each independently represent a substituted or unsubstituted aromatic heterocyclic group (except for a carbazolyl group, a phenoxazyl group, and a phenothiazyl group) and may be a substituent in which an aromatic hydrocarbon group and an aromatic heterocyclic group are combined; o, p, q, and r are each independently 1 or 2; Ar₃-Ar₆ each independently represent a divalent or trivalent aromatic hydrocarbon group, and said hydrocarbon group may each be independently substituted by hydrogen atom, an alkyl group, a cycloalkyl group, or an alkoxy group; and a ring E₁ and a ring E₂ represent a pyridine ring and may each independently have a substituent selected from hydrogen atom, an alkyl group, a cycloalkyl group, and an alkoxy group.)

5. The bipyridine derivative as claimed in claim 4, being expressed by the following general formula (5). 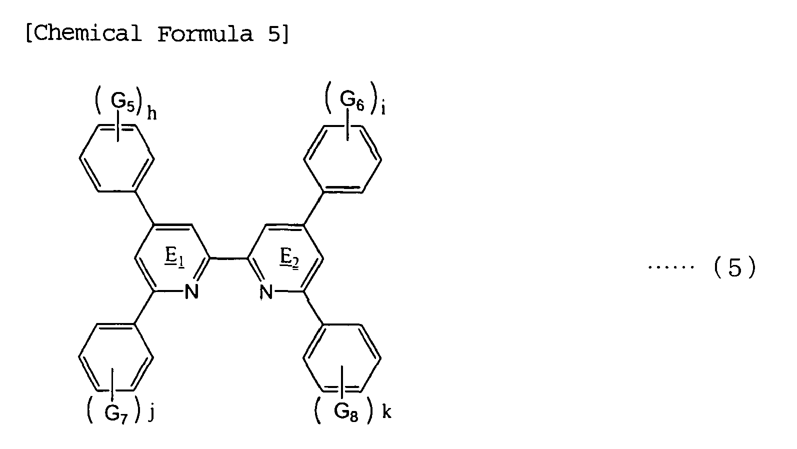 (where G₅ - G₈ each independently represent a substituted or unsubstituted aromatic heterocyclic group (except for a carbazolyl group, a phenoxazyl group, and a phenothiazyl group) and may be a substituent in which an aromatic hydrocarbon group and an aromatic heterocyclic group are combined; any hydrogen atom on benzene ring may be substituted by an alkyl group, a cycloalkyl group, or an alkoxy group; h, i, j, and k are each independently 1 or 2; and a ring E₁ and a ring E₂ represent a pyridine ring and may each independently have a substituent selected from hydrogen atom, an alkyl group, a cycloalkyl group, and an alkoxy group.)

6. A bipyridine derivative expressed by the following general formula (6). 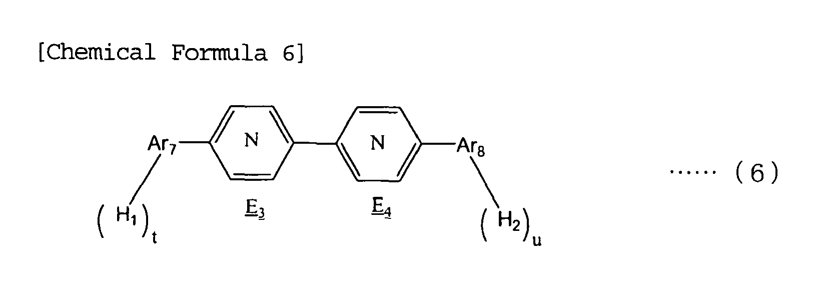 (where H₁ and H₂ are each independently represent a substituted or unsubstituted aromatic heterocyclic group (except for a carbazolyl group) and may be a substituent in which an aromatic hydrocarbon group and an aromatic heterocyclic group are combined; Ar₇ and Ar₈ each independently represent a divalent or trivalent aromatic hydrocarbon group, and said hydrocarbon group may each be independently substituted by hydrogen atom, an alkyl group, a cycloalkyl group, or an alkoxy group; t and u are each independently 1 or 2; and a ring E₃ and a ring E₄ represent a pyridine ring, and may each independently have a substituent selected from hydrogen atom, an alkyl group, a cycloalkyl group, and an alkoxy group.)

7. The bipyridine derivative as claimed in claim 6, being expressed by the following general formula (7). 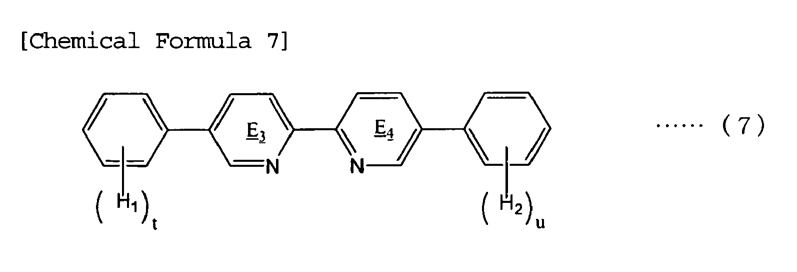 (where H₁ and H₂ each independently represent a substituted or unsubstituted aromatic heterocyclic group (except for a carbazolyl group) and may be a substituent in which an aromatic hydrocarbon group and an aromatic heterocyclic group are combined; any hydrogen atom on benzene ring may be substituted by an alkyl group, a cycloalkyl group, or an alkoxy group; t and u are each independently 1 or 2; and a ring E₃ and a ring E₄ represent a pyridine ring, and may each independently have a substituent selected from hydrogen atom, an alkyl group, a cycloalkyl group, and an alkoxy group.)

8. A bipyridine derivative expressed by the following general formula (8). 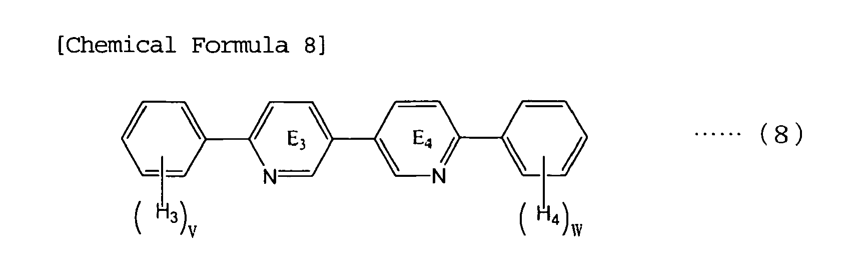 (where H₃ and H₄ each independently represent a substituted or unsubstituted aromatic hydrocarbon group or a substituted or unsubstituted aromatic heterocyclic group and may be a substituent in which an aromatic hydrocarbon group and an aromatic heterocyclic group are combined; any hydrogen atom on benzene ring may be substituted by an alkyl group, a cycloalkyl group, or an alkoxy group; v and w are each independently 1 or 2; and a ring E₃ and a ring E₄ may each independently have a substituent selected from hydrogen atom, an alkyl group, a cycloalkyl group, and an alkoxy group.)

9. The bipyridine derivative as claimed in any one of claims 1-8, **characterized by** being used as an organic electronics element material.

10. The bipyridine derivative as claimed in any one of claims 1-8, **characterized by** being used as an electron transport material.

11. An organic electroluminescence element having at least one organic layer between a pair of electrodes, wherein at least one layer of said organic layers contains the bipyridine derivative as claimed in any one of claims 1-8.

12. An organic electroluminescence element, wherein at least one of an electron transport layer, an electron injection layer, a hole inhibition layer, and a light emitting layers contains the bipyridine derivative as claimed in any one of claims 1-8.
